# EUROPEAN PATENT APPLICATION

(11) **EP 1 421 957 A1**
(43) Date of publication of application: **26.05.2004**
(21) Application number: 03252184.1
(22) Date of filing: 07.04.2003
(51) Int. Cl.: A61L 27/38, A61L 27/42, A61F 2/02, A61F 2/08, A61F 2/10

(54) **Treatment of tissue degeneration and replacement of tissue with undifferentiated mesenchymal cells**

(30) Priority: 21.11.2002 US 301058
(71) Applicant: Isolagen International S.A., 2000 Neuchâtel (CH)
(72) Inventor: Marko, Olga, Paramus, New Jersey 07652 (US); Boss, William K., Jr., Essex Fells, New Jersey 07021 (US)
(74) Representative: Brady, Paul Andrew

(57) **Abstract**

The present invention provides compositions and methods for correcting cosmetic, aesthetic, and degenerative defects in the skin, soft tissue, and bone of a subject. In particular, methods of the invention involve the injection or implantation of autologous UMC, fibroblasts, and/or kcratinocytes into the tissue (e.g., subcutaneous tissue) adjacent or subadjacent to a defect or at the site of a defect. The cells that are injected, as provided herein, are histocompatible with the subject (e.g., are autologous) and have been expanded by passage in a cell culture system.

## Description

### TECHNICAL FIELD

This invention relates to compositions containing undifferentiated mesenchymal cells and methods for treating dental defects, defects of skin and soft tissue, and bone defects with autologous undifferentiated mesenchymal cells.

### BACKGROUND

Defects of the skin, bone, soft tissue, and dental tissue can be caused by factors such as wounds, disease, and aging. Methods have been designed to repair and/or augment tissue that is affected by such conditions, but improved methods that avoid scarring and potentiate tissue growth would be useful.

U.S. Patent Nos. 5,591,444; 5,660,850; 5,665,372; and 5,858,390; and copending U.S. application Serial No. 09/678,047 are incorporated herein by reference in their entirety.

### SUMMARY

The present invention provides compositions and methods for correcting cosmetic, aesthetic, and degenerative defects in the skin, soft tissue and bone of a subject. In particular, methods of the invention involve the injection or implantation of autologous, undifferentiated mesenchymal cells (UMC), fibroblasts, and/or keratinocytes into the tissue (e.g., intradermal tissue) adjacent or subadjacent to a defect or at the site of a defect. Defects that can be corrected by this method include, for example, rhytids, stretch marks, depressed scars, cutaneous depressions of non-traumatic origin, scarring from acne vulgaris, hypoplasia of the lip, periodontal defects, soft tissue defects such as velopharyngeal incompetence and subcutaneous atrophy, bone defects, and bums. The cells that are injected, as provided herein, are histocompatible with the subject and have been expanded by passage in a cell culture system. The injected cells preferably are autologous cells. The invention also features an in vitro method of generating a population of cells that includes UMC.

In one embodiment, the composition can include both passaged, autologous UMC and passaged, autologous fibroblasts. Such cells can be derived, for example, from the culture of one or more biopsy specimens taken from the subject. In another embodiment, the composition can include passaged, autologous UMC with a biodegradable acellular matrix, with or without passaged, autologous fibroblasts. In yet another embodiment, the composition can include passaged, autologous UMC together with a biodegradable acellular filler, with or without passaged, autologous fibroblasts. Other embodiments contain passaged, autologous keratinocytes and passaged, autologous fibroblasts, with or without matrices or fillers.

The invention further provides methods for rendering the passaged autologous UMC, fibroblasts, and keratinocytes substantially free of immunogenic proteins present in the culture medium (i.e., culture medium serum-derived proteins), so that the cells can be used to correct defects in a subject without stimulating an immune response. The method involves incubating the expanded UMC, fibroblasts, keratinocytes, or biodegradable acellular matrices containing one or more of these cell populations in serum-free medium for a period of time subsequent to culturing in serum (e.g., fetal bovine serum (FBS)) containing medium. In another embodiment, the cells or biodegradable acellular matrices containing cells are cultured only in serum-free medium or in medium containing autologous serum.

The present invention is based, in part, on the discovery that autologous cells are an ideal material with which to augment tissue such as the dermis, subcutaneous tissue, and bone, at sites that are at or near (e.g., subadjacent to) a defect. The invention also is based on the recognition that an abundant supply of autologous cells can be obtained by culturing a biopsy specimen taken from a subject prior to treatment of the defect. The invention is further based on the recognition that, after expansion in xenogeneic serum-containing tissue culture medium, autologous cells contain a significant quantity of immunogenic proteins derived from the xenogeneic serum, but that the immunogenic proteins can be removed prior to injection into the subj ect.

In one aspect, the invention features a method for making a cellular composition for repairing tissue; the method involves: (a) providing a biopsy of undifferentiated mesenchymal cell (UMC)-containing tissue to obtain starting cells; (b) separating starting cells from said biopsy; (c) culturing the starting cells; and (d) harvesting a population of non-adherent derivative cells from the culture, the non-adherent derivative cells containing UMC. The method can further include one or more (e.g., one, two, three, four, five, six, seven, eight, nine, ten, 11, 12, 13, 14, 15, 16, 17, 18, 19, 20, 25, 30, 35, 40, 45, 50, 55, 60, 65, 70, 75, 80, 90, 95, 100, or more) rounds of derivatization that includes repeating steps (c) and (d) utilizing the population of harvested non-adherent derivative cells from the previous round as the starting cells. The one or more additional rounds of derivatization can include, for example, from one to twenty rounds. The UMC-containing tissue can be selected from the group consisting of: dermal tissue, adipose tissue, connective tissue, fascia, lamina propria and bone marrow. The method can further involve culturing the non-adherent cells in the presence of acidic fibroblast growth factor (aFGF).

In another aspect, the invention features a composition for repairing tissue. The composition can contain autologous, passaged UMC, and can be substantially free of culture medium serum-derived proteins. The composition can further contain autologous, passaged fibroblasts. The autologous fibroblasts can be from gums, palate, skin, lamina propria, connective tissue, bone marrow, fascia, or adipose tissue of the subject. The UMC or the fibroblasts can be treated with an activating compound (e.g., ascorbyl palmitate, linoleic acid, C-MED 100® (Optigene-X LLC, Shrewsbury, NJ), quinic acid, quinic acid salts, quinic lactones, CoEnzyme Q-10, L-hydroxy acid, L-lipoic acid, calcium monophosphate, calcium triphosphate, niacin, dehydroepiandrosterone (DHEA), dimethylamino ethanol (DMAE), α-tocopherol, bone morphogenic proteins (BMP), vitamin E, vitamin C, carotenoids, glycolic acid, carboxyalkyl esters, estriol, acetyl-L-carnitine, deprenyl, lycopene, nicotinamide adenine dinucleotide (NADH), cysteine, procysteine, pikamilon, vinpocetine, retinoic acid, antineoplastons, or other stimulatory additives such as growth factors [e.g., human growth hormone, fibroblast growth factor (FGF), vascular endothelial growth factor (VEGF), insulin-like growth factor-I (IGF-I), insulin, and long form R3 IGF-I]). The UMC or the fibroblasts can also be exposed to a low energy laser light prior to administration to a subject. The composition can further contain a biodegradable acellular matrix, wherein the UMC and/or fibroblasts are integrated within or on the matrix. The matrix, prior to combination with the UMC and/or fibroblasts, can contain one or more substances selected from the group consisting of: collagen (e.g., bovine collagen, porcine collagen, human collagen, engineered collagen, or collagen and glycosaminoglycans cross-linked with glutaraldehyde), glycosaminoglycans, gelatin, polyglycolic acid, cat gut, demineralized bone, hydroxyapatite, hyaluron, hyaluronic acid, coral, and anorganic bone. Sufficient UMC and/or fibroblasts can integrate on and within the matrix to substantially fill the space on or within the matrix available for cells. The composition can also or alternatively contain a biodegradable acellular injectable filler. The biodegradable acellular injectable filler, prior to combination with the UMC and/or fibroblasts, can include one or more substances selected from the group consisting of: (a) an injectable dispersion of autologous collagen fibers; (b) collagen (e.g., bovine collagen, reconstituted bovine collagen fibers cross-linked with glutaraldehyde, or autologous collagen); (c) solubilized gelatin; (d) solubilized polyglycolic acid; (e) solubilized cat gut; (f) porcine gelatin powder and amino caproic acid dispersed in sodium chloride solution and an aliquot of plasma from the subject; and (g) hyaluronic acid.

In another aspect, the invention features a method for making a composition for repairing tissue that has degenerated in a subject as a result of a disease, disorder, or defect in the subject. The method can involve: (a) providing a biopsy of UMC-containing tissue from the subject; (b) separating autologous UMC from the biopsy; (c) culturing the autologous UMC under conditions that produce autologous UMC that are substantially free of culture medium serum-derived proteins; and (d) exposing the cultured autologous UMC to conditions that result in suspension of the UMC. The UMC-containing tissue can be selected from the group consisting of: gums, palate, skin, lamina propria, connective tissue, fascia, bone marrow, and adipose tissue. Culturing of the UMC can involve: (1) incubation in a culture medium containing between 0.1 % and about 20% human or non-human serum, followed by (2) incubation in a serum-free culture medium. Alternatively, the culturing of the UMC can be only in serum-free medium. The conditions that result in suspension of the UMC can include the use of a proteolytic enzyme, for example trypsin. Alternative proteolytic enzymes include dispase and collagenase. The conditions that result in suspension of the UMC can additionally or alternatively include the use of EDTA. The method can further involve: (e) providing a biopsy of fibroblast-containing tissue from the subject; (f) separating autologous fibroblasts from the biopsy; (g) culturing the autologous fibroblasts under conditions that produce fibroblasts that are substantially free of culture medium serum-derived proteins; (h) exposing the cultured autologous fibroblasts to conditions that result in suspension of the fibroblasts; and (i) mixing the UMC suspension with the fibroblast suspension. The autologous fibroblast-containing tissue can be selected from the group consisting of: gums, palate, skin, lamina propria, connective tissue, fascia, bone marrow, and adipose tissue. Culturing of the fibroblasts can involve: (1) incubation in a culture medium containing between 0.1 % and about 20% human or non-human serum, followed by (2) incubation in a serum-free culture medium. Alternatively, the culturing of the fibroblasts can be only in serum-free medium. The conditions that result in suspension of the fibroblasts can include the use of a proteolytic enzyme, for example trypsin. Alternative proteolytic enzymes include dispase and collagenase. The conditions that result in suspension of the UMC can additionally or alternatively include the use of EDTA. Prior to combining, the UMC or the fibroblasts can be exposed to an activating compound (e.g., ascorbyl palmitate, linoleic acid, C-MED 100® , quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, BMP, vitamin E, vitamin C, carotenoids, glycolic acid, carboxyalkyl esters, estriol, acetyl-L-carnitine, deprenyl, lycopene, NADH, cysteine, procysteine, pikamilon, vinpocetine, retinoic acid, antineoplastons, or other stimulatory additives such as growth factors [e.g., human growth hormone, FGF, VEGF, IGF-I, insulin, and long form R3 IGF-I]). The UMC or the fibroblasts can be exposed to a low energy laser light.

In yet another aspect, the invention features a method for making a composition for repairing tissue that has degenerated in a subject as a result of a disease, disorder, or defect in the subject. The method can involve: (a) providing autologous, passaged UMC; (b) providing a biodegradable acellular matrix; and (c) incubating the UMC with the biodegradable acellular matrix such that the UMC integrate on or within the biodegradable acellular matrix, wherein the incubation results in a composition for repairing tissue, and wherein the conditions of the incubation are such that the composition is substantially free of culture medium serum-derived proteins. The step of providing a suspension of autologous, passaged UMC can involve: (a) providing a biopsy of UMC-containing tissue from the subject; (b) separating autologous UMC from the biopsy; (c) culturing the UMC; and (d) exposing the cultured UMC to conditions that result in suspension of the UMC. The UMC-containing tissue can be selected from the group consisting of: gums, palate, skin, lamina propria, connective tissue, fascia, bone marrow, and adipose tissue. The biodegradable acellular matrix, prior to combination with the suspension of UMC, can contain one or more substances selected from the group consisting of: collagen (e.g., collagen and glycosaminoglycans, cross-linked with glutaraldehyde, bovine collagen, or porcine collagen), glycosaminoglycans, gelatin, polyglycolic acid, cat gut, demineralized bone, hydroxyapatite, coral, and anorganic bone. The incubation conditions can include: (1) culturing in culture medium containing between 0.1 % and about 20% human or non-human serum, followed by (2) culturing in serum-free culture medium. Alternatively the culturing can be only in serum-free medium. Sufficient UMC can integrate within the biodegradable acellular matrix to substantially fill the space on or within the biodegradable acellular matrix available for cells.

The method can further involve providing autologous, passaged fibroblasts, incubating the fibroblasts with the biodegradable acellular matrix such that the fibroblasts integrate on or within the biodegradable acellular matrix, wherein the incubation results in a composition for repairing tissue, and wherein the conditions of the incubation are such that the composition is substantially free of culture medium serum-derived proteins. The step of providing autologous, passaged fibroblasts can involve: (a) providing a biopsy of fibroblast-containing tissue from the subject; (b) separating autologous fibroblasts from the biopsy; (c) culturing the fibroblasts; and (d) suspending the fibroblasts. The fibroblast-containing tissue can be selected from the group consisting of: gums, palate, skin, lamina propria, connective tissue, fascia, bone marrow, and adipose tissue. Sufficient UMC and fibroblasts can integrate on or within the biodegradable acellular matrix to substantially fill the space on or within the biodegradable acellular matrix available for cells. Prior to the incubating with the biodegradable acellular matrix, the UMC and/or the fibroblasts can be exposed to an activating compound (e.g., ascorbyl palmitate, linoleic acid, C-MED 100® , quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, BMP, vitamin E, vitamin C, carotenoids, glycolic acid, carboxyalkyl esters, estriol, acetyl-L-camitine, deprenyl, lycopene, NADH, cysteine, procysteine, pikamilon, vinpocetine, retinoic acid, antineoplastons, or other stimulatory additives such as growth factors [e.g., human growth hormone, FGF, VEGF, IGF-I, insulin, and long form R3 IGF-I]). The UMC or the fibroblasts can be exposed to a low energy laser light. The UMC and the fibroblasts can be together incubated with the biodegradable acellular matrix, or the UMC and the fibroblasts are added separately to the biodegradable acellular matrix.

In another aspect, the invention features a method for making a composition for repairing tissue that has degenerated in a subject as a result of a disease, disorder, or defect in the subject. The method can involve: (a) providing autologous, passaged UMC, wherein the UMC are substantially free of culture medium serum-derived proteins; (b) providing a biodegradable acellular filler; and (c) combining the autologous, passaged UMC and the biodegradable acellular filler. The step of providing a suspension of autologous, passaged UMC can involve: (a) providing a biopsy of UMC-containing tissue from the subject; (b) separating autologous UMC from the biopsy; (c) culturing the autologous UMC under conditions that result in UMC that are substantially free of culture medium serum-derived proteins; and (d) exposing the cultured UMC to conditions that result in suspension of the UMC. The UMC-containing tissue can be selected from the group consisting of: gums, palate, skin, lamina propria, connective tissue, fascia, bone marrow, and adipose tissue. Culturing of the UMC can involve: (1) culturing in a medium containing between 0.1% and about 20% human or non-human serum, followed by (2) culturing in a serum-free medium. Culturing of the UMC can involve culturing in serum-free medium. The conditions that result in suspension of the UMC can include the use of a proteolytic enzyme, for example trypsin. Alternative proteolytic enzymes include dispase and collagenase. The conditions that result in suspension of the UMC can additionally or alternatively include the use of EDTA. The biodegradable acellular filler, prior to combination with the UMC, can contain one or more substances selected from the group consisting of: (a) an injectable dispersion of autologous collagen fibers; (b) collagen (e.g., bovine collagen, reconstituted bovine collagen fibers cross-linked with glutaraldehyde, or autologous collagen); (c) solubilized gelatin; (d) solubilized polyglycolic acid; (e) solubilized cat gut; (f) porcine gelatin powder and amino caproic acid dispersed in sodium chloride solution and an aliquot of plasma from the subject; and (g) hyaluronic acid.

The method can further involve providing autologous, passaged fibroblasts, wherein the autologous, passaged fibroblasts are substantially free of culture medium serum-derived proteins, and combining the autologous, passaged fibroblasts and the biodegradable acellular filler. The step of providing a suspension of autologous, passaged fibroblasts can involve: (a) providing a biopsy of fibroblast-containing tissue from the subject; (b) separating autologous fibroblasts from the biopsy; (c) culturing the autologous fibroblasts under conditions that result in fibroblasts that are substantially free of culture medium serum-derived proteins; and (d) exposing the incubated autologous fibroblasts to conditions that result in suspension of the fibroblasts. The fibroblast-containing tissue can be selected from the group consisting of: gums, palate, skin, lamina propria, connective tissue, fascia, bone marrow, and adipose tissue. Culturing of the fibroblasts can involve: (1) culturing in a medium containing between 0.1 % and about 20% human or non-human serum, followed by (2) culturing in a serum-free medium. Alternatively the culturing of the fibroblasts can be only in serum-free medium. The conditions that result in suspension of the fibroblasts can include bringing the fibroblasts into contact with a proteolytic enzyme, for example trypsin. Alternative proteolytic enzymes include dispase and collagenase. The conditions that result in suspension of the fibroblasts can additionally or alternatively include the use of EDTA. Prior to the combining with the UMC and the biodegradable acellular filler, the UMC or the fibroblasts can be exposed to an activating compound (e.g., ascorbyl palmitate, linoleic acid, C-MED 100® , quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, BMP, vitamin E, vitamin C, carotenoids, glycolic acid, carboxyalkyl esters, estriol, acetyl-L-camitine, deprenyl, lycopene, NADH, cysteine, procysteine, pikamilon, vinpocetine, retinoic acid, antineoplastons, or other stimulatory additives such as growth factors [e.g., human growth hormone, FGF, VEGF, IGF-I, insulin, and long form R3 IGF-I]). The UMC or the fibroblasts can be exposed to a low energy laser light.

In still another aspect, the invention features a method for repairing tissue in a subject. The method can involve: (a) providing a composition containing autologous, passaged UMC, wherein the composition is substantially free of culture medium serum-derived proteins; (b) identifying a site of tissue defect or tissue degeneration in the subject; and (c) placing the composition at the site so that the tissue defect or degeneration is repaired. The tissue defect or tissue degeneration can include a soft tissue defect, a defect of an oral mucosa, trauma to an oral mucosa, periodontal disease, a bone defect, diabetes, a cutaneous ulcer, venous stasis, or a cosmetic defect of the skin. The soft tissue defect can be selected from the group consisting of a facial depression, underdevelopment of the breast, absence of breast, breast reconstruction, a vocal cord defect, velopharyngeal incompetence, Poland's syndrome, underdevelopment of male genitalia (e.g., penis), hypoplasia of the lips, and a subcutaneous atrophy or muscular atrophy. The trauma to an oral mucosa can be an extraction socket resulting from an extraction of a tooth. The periodontal disease can involve periodontal degeneration, gingivitis, or non-healing wounds of a palatal mucosa or a gingival mucosa. The bone defect can be selected from the group consisting of hemifacial microsomia, posttraumatic non-healing of a bone, enophthalmos, Romberg's disease, cranial burr hole defects, loss of skull bone, a defect that result from a blunt trauma, and a defect that results from arthritis. The cosmetic defect can be selected from the group consisting of: a rhytid, a depressed scar, a cutaneous depression of non-traumatic origin, a laugh line, a stretch mark, a wrinkle, a wound scar, an acne scar, and subcutaneous atrophy. The tissue defect can be hypoplasia of a lip or a lip fold. The autologous UMC can be from gums, palate, skin, lamina propria, connective tissue, fascia, bone marrow, or adipose tissue of the subject. The composition can further comprise a biodegradable matrix, wherein the UMC are integrated within or on the matrix. The composition also can comprise a biodegradable acellular filler. The composition can further contain autologous, passaged fibroblasts.

In another aspect, the invention provides a method for repairing tissue in a subject. The method can involve: (a) providing a composition containing autologous, passaged UMC and autologous, passaged fibroblasts, wherein the composition is substantially free of culture medium serum-derived proteins; (b) identifying a site of tissue defect or tissue degeneration in the subject; and (c) placing the composition at the site so that the tissue defect or degeneration is repaired.

In another aspect, the invention features a method for repairing a tissue defect in a subject. The method can involve: (a) providing a pharmaceutical composition containing: (1) autologous, passaged UMC, (2) autologous, passaged fibroblasts, and (3) a pharmaceutically acceptable carrier thereof; wherein the pharmaceutical composition is substantially free of culture medium serum-derived proteins; (b) identifying in the subject a site of tissue defect or tissue degeneration selected from the group consisting of: a dental or periodontal defect, a cosmetic defect of the skin, and a bone defect; and (c) injecting a therapeutically effective amount of the pharmaceutical composition adjacent to the site of tissue defect or degeneration, wherein the injecting results in repair of the tissue defect or degeneration. The UMC-containing and fibroblast-containing tissues can be selected from the group consisting of: gums, palate, skin, lamina propria, connective tissue, fascia, bone marrow, and adipose tissue. The UMC or the fibroblasts can have been: (1) cultured in a medium containing between 0.1 % and about 20% human or non-human serum, and then (2) cultured in a serum-free medium. The UMC or the fibroblasts can have been cultured in serum-free medium.

In another aspect, the invention features a method for repairing tissue that has degenerated in a subject as a result of a disease, disorder, or defect in the subject. The method can involve placing a composition into the subject at the site of degeneration so that the tissue is repaired. The composition can contain (1) autologous, passaged UMC, (2) autologous, passaged fibroblasts, and (3) a biodegradable matrix, wherein the UMC or the fibroblasts are integrated within or on the matrix, and wherein the composition is substantially free of culture medium serum-derived proteins.

The invention also features a method for repairing tissue that has degenerated in a subject as a result of a disease, disorder, or defect in the subject. The method can involve injecting a composition containing (1) autologous, passaged UMC that are substantially free of culture medium serum-derived proteins and (2) a biodegradable acellular injectable filler into the subject at the site of the degeneration so that the tissue is repaired. The composition further can further contain autologous, passaged fibroblasts.

In another aspect, the invention features a method for repairing tissue that has degenerated in a subject as a result of a disease, disorder, or defect in the subject. The method can include the steps of: (a) injecting autologous, passaged UMC into the subject at a site of tissue degeneration, wherein the UMC are substantially free of culture medium serum-derived proteins; (b) injecting autologous, passaged fibroblasts into the subject at a site of a tissue defect or desired tissue augmentation, wherein the fibroblasts are substantially free of culture medium serum-derived proteins; and (c) injecting a biodegradable, acellular filler into the site. The disease, disorder, or defect can be a defect selected from the group consisting of: a dental or periodontal defect, a cosmetic defect of the skin, and a bone defect. The autologous UMC and fibroblasts can be from gums, palate, skin, lamina propria, connective tissue, fascia, bone marrow, or adipose tissue of the subject. The UMC and the fibroblasts can be injected simultaneously. The UMC, the fibroblasts, and the biodegradable acellular filler can be injected simultaneously. The UMC and the fibroblasts can be injected separately. The UMC and the fibroblasts can be injected separately from the biodegradable acellular filler. The duration between injecting the UMC and the fibroblasts into the subject and injecting the biodegradable acellular filler into the subject can be about two weeks

In another aspect, the invention features a method for repairing a burn wound in a subject. The method can involve providing a suspension of autologous, passaged keratinocytes and autologous, passaged fibroblasts, and injecting the suspension into the bum wound. Providing a suspension of autologous, passaged keratinocytes and autologous, passaged fibroblasts can involve: (a) providing a biopsy of keratinocyte-containing tissue from the subject; (b) separating autologous keratinocytes from the biopsy; (c) culturing the autologous keratinocytes under conditions that produce autologous keratinocytes that are substantially free of culture medium serum-derived proteins; (d) exposing the keratinocytes to conditions that result in suspension of the keratinocytes; (e) providing a biopsy of fibroblast-containing tissue from the subject; (f) separating autologous fibroblasts from the biopsy; (g) culturing the autologous fibroblasts under conditions that produce autologous fibroblasts that are substantially free of culture medium serum-derived proteins; (h) exposing the fibroblasts to conditions that result in suspension of the fibroblasts; and (i) combining the suspended keratinocytes and the suspended fibroblasts. The keratinocyte-containing tissue can be selected from the group consisting of: skin, oral mucosa, gums, buccal tissue, esophageal tissue, exocervical tissue, and conjunctival tissue. Culturing of the keratinocytes can involve (1) incubation in a culture medium containing between 0.1 % and about 20% human or non-human serum, followed by (2) incubation in a serum-free culture medium. Alternatively the culturing of the keratinocytes can be only in serum-free medium. The fibroblast-containing tissue can be selected from the group consisting of: gums, palate, skin, lamina propria, connective tissue, fascia, bone marrow, and adipose tissue. Culturing of the fibroblasts can involve: (1) incubation in a culture medium containing between 0.1 % and about 20% human or non-human serum, followed by (2) incubation in a serum-free culture medium. Alternatively, the culturing of the fibroblasts can be only in serum-free medium. The conditions that result in suspension of the fibroblasts can include bringing the fibroblasts into contact with a proteolytic enzyme, for example trypsin. Alternative proteolytic enzymes include dispase and collagenase. The conditions that result in suspension of the fibroblasts can additionally or alternatively include the use of EDTA. Prior to combining with the keratinocytes, the fibroblasts can be exposed to an activating compound (e.g., ascorbyl palmitate, linoleic acid, C-MED 100® , quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, BMP, vitamin E, vitamin C, carotenoids, glycolic acid, carboxyalkyl esters, estriol, acetyl-L-camitine, deprenyl, lycopene, NADH, cysteine, procysteine, pikamilon, vinpocetine, retinoic acid, antineoplastons, or other stimulatory additives such as growth factors [e.g., human growth hormone, FGF, VEGF, IGF-I, insulin, and long form R3 IGF-1]). The fibroblasts can also be exposed to a low energy laser light prior to administration to a subject. The suspension can contain autologous keratinocytes and autologous fibroblasts in a ratio of about 1:3. The injecting can involve injecting the suspension into the central region of the bum wound. The suspension can further contain autologous, passaged UMC. The method can further include injecting an enhancing compound into the wound.

The invention further provides the use of undifferentiated mesenchymal cells (UMCs) for the manufacture of a medicament for use in the treatment of tissue degeneration or a tissue defect selected from soft tissue defect, a defect of an oral mucosa, trauma to an oral mucosa, periodontal disease, diabetes, a cutaneous ulcer and venous stasis, in which the medicament comprises autologous, passaged UMCs substantially free of culture medium serum-derived proteins. Preferably, the composition is substantially free of cells other than passaged autologous UMC, passaged, autologous fibroblasts and, optionally, passaged, autologous keratinocytes.

The invention further provides the use of undifferential mesenchymal cells for the manufacture of a medicament for simultaneous, sequential or separate administration of for use in the repair of tissue that has degenerated in a subject as a result of a disease, disorder, or defect in said subject, wherein said UMC are passaged UMC substantially free of culture medium serum-derived proteins; and said fibroblasts are autologous, passaged fibroblasts into substantially free of culture medium serum-derived proteins.

The invention also provides a composition for use as a medicament, wherein said composition comprises autologous, passaged UMC and autologous, passaged fibroblasts, and wherein said composition is substantially free of culture medium serum-derived proteins. A composition for the cosmetic repair of tissue, wherein said composition comprises autologous, passaged UMC and autologous, passaged fibroblasts, and wherein said composition is substantially free of culture medium serum-derived proteins is also provided.

The invention also provides the use of autologous, passaged keratinocytes and autologous, passaged fibroblasts, for the manufacture of a medicament for use in the repair of a bum wound in a subject.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description below. Other features, objects, and advantages of the invention will be apparent from the description and from the claims.

### DETAILED DESCRIPTION

The invention provides compositions and methods for treating disorders or defects that result in degeneration of tissue in a subject. Such disorders or defects include defects of the oral mucosa, trauma to the oral mucosa (e.g., an extraction socket that can result from extraction of a tooth), trauma to oral bones such as the maxillary or mandibular bones, periodontal disease, diabetes, cutaneous ulcers, or venous stasis. Examples of periodontal disease that can result in tissue degeneration include, but are not limited to, periodontal degeneration, gingivitis, or non-healing wounds of the palatal mucosa or gingival mucosa, or bone degeneration. Other defects that can be treated using the compositions and methods provided herein include, without limitation, skin defects such as scars, wrinkles, laugh lines, stretch marks, depressed scars, cutaneous depressions of non-traumatic origin, acne scarring or subcutaneous atrophy from acne, trauma, congenital malformation, or aging. Moreover, compositions and methods of the invention can be used to treat defects such a hypoplasia of the lips, labial folds, or bone defects, e.g., defects of bones such as, for example, facial bones including orbits, zygomatic bones, crania, cleft palate bone defects, and nasal bones, as well as spinal bones and long bones. Bone defects include, for example, hemifacial microsomia, posttraumatic non-healing of any bone (e.g., a bone of the hand, wrist, leg, spine, or face), enophthalmos, Romberg's disease, cranial burr hole defects, and loss of skull bone. Compositions and methods of the invention also can be used to aid in healing after surgery on any bone. The invention also can be used to augment soft tissue defects, including facial depressions, underdeveloped breasts, absence of breasts, vocal cord defects, velopharyngeal incompetence, Poland's syndrome, and any subcutaneous atrophies or muscular atrophies including post poliomyelitis atrophy and hypoplasia of the buttocks, calves, or male genitalia.

### 1. Compositions containing cells

The invention provides compositions containing cells such as, for example, autologous, passaged UMC, autologous, passaged fibroblasts from any source, and/or autologous, passaged keratinocytes. Compositions of the invention are substantially free of immunogenic proteins (e.g., culture medium serum-derived proteins). As used herein, the term "autologous" as applied to components of compositions administered to recipients refers to body components (e.g., cells or biological molecules such as proteins, nucleic acids, carbohydrates or lipids) removed from a donor and administered to a recipient, wherein the donor and recipient are the same individual. As used herein, cells that are "substantially free of culture medium serum-derived proteins" are cells in which the fluid into which the cells are incorporated contains less than 0.1% (e.g., less than 0.05%, less than 0.01%, less than 0.005%, or less than 0.001 %) of xenogeneic or allogeneic serum contained in the tissue culture medium in which the cells were cultured. Similarly, a composition that is "substantially free of culture medium serum-derived proteins" is a composition in which fluid surrounding the cells in the composition contains less than 0.1% (e.g., less than 0.05%, less than 0.01%, less than 0.005%, or less than 0.001%) of xenogeneic or allogeneic serum contained in the tissue culture medium in which the cells were cultured.

To obtain cells that are substantially free of culture medium serum-derived proteins, cultured cells can be expanded in medium that does not contain serum. Alternatively, cells can be cultured first in medium that contains serum (e.g., 0.1% to 20% serum), and subsequently cultured in serum-free medium. The presence of potentially immunogenic serum-derived proteins in a cell suspension is thus avoided by these methods.

The cells used herein can be from any mammalian species (e.g., humans, non-human primates, dogs, cows, horses, pigs, sheep, cats, rabbits, mice, rats, guinea pigs, hamsters, or gerbils) provided that the cells are autologous. Autologous human cells (e.g., autologous human UMC, fibroblasts, and keratinocytes) are particularly useful. It is noted that when animals are inbred and are thus isogenic (e.g., as in some inbred laboratory strains of animals such as rats and mice), "autologous" can mean derived from another individual of the same species. The cells used herein also can be non-autologous (e.g., obtained from a subject other than the recipient, or from a cell line).

The compositions provided herein typically are prepared by obtaining a tissue biopsy from the subject, separating the desired cells from the biopsy, culturing the cells through a suitable number of passages to obtain the desired quantity of cells, and suspending the cells. As used herein, "adherent" cells are cells that adhere to the material (e.g., plastic) of a standard tissue culture vessel. As used herein, "non-adherent" cells include cells that do not adhere to the material (e.g., plastic) of a standard tissue culture vessel, as well as cells that detach from the surface of a tissue culture vessel when space and nutrients become limiting. Once such cells are harvested and placed into suitable conditions, they can attach and expand. Compositions thus can contain any cell that can be expanded in culture. UMC and fibroblasts can be isolated from, for example, dermal tissue or adipose tissue. UMC and fibroblasts also can be obtained from, without limitation, fascia, lamina propria, the bulbar area of hair follicles, bone marrow, or any source of connective tissue. Autologous keratinocytes can be obtained from, for example, a full-thickness skin biopsy of the subject, from scalp, or from hair follicles. Adherent and non-adherent cells can be substantially separated from each other by differential proteolysis, for example trypsinization, and enrichment or by gradient purification and enrichment using Ficoll or Percoll. Ficoll or Percoll are available, amongst others from Amersham Biosciences (Amersham Place, Little Chalfont, Buckinghamshire, HP7 9NA, UK).

Due to the phenomenon of allograft (or xenograft) rejection, which is well known to those in the art, it is preferred that the cultured cells be histocompatible with the recipient. Histocompatibility can be ensured by obtaining cells (e.g., peripheral blood mononuclear cells) from the subject to be treated and determining the major histocompatibility complex (MHC) haplotype of the subject using such cells. It is understood, however, that such cells also can be obtained from an identical twin of the subject, or from an individual that is identical at the MHC with the subject. Cells isolated from a biopsy specimen can be cultured such that they are substantially free of culture medium serum-derived proteins. This step reduces the ability of the cells to activate an untoward immune response in the subject.

Before initiation of the cell culture, a biopsy can be washed repeatedly with antibiotic and antifungal agents in order to reduce the potential for contamination of subsequence cultures. A suitable "wash medium" can contain, for example, tissue culture medium such as Dulbecco's Modified Eagle's Medium (DMEM), Iscove's Modified Dulbecco's Medium (IMDM), or any suitable culture medium, along with some or all of the following agents: gentamicin, amphotericin B (FUNGIZONE® ), Mycoplasma removal agent (MRA; Dianippon Pharmaceutical Company, Japan), plasmocin, and tylosin (available from, for example, Serva, Heidelberg, Germany). Gentamicin can be used at a concentration of 10 to 100 *µ*g/ml (e.g., 25 to 75 *µ*g/ml, or about 50 *µ*g/ml). Amphotericin B can be used at a concentration of 0.5 to 12.5 *µ*g/ml (e.g., 1.0 to 10.0 *µ*g/ml, or about 2.5 *µ*g/ml). MRA can be used at a concentration of 0.1 to 1.5 *µ*g/ml (e.g., 0.25 to 1.0 *µ*g/ml, or about 0.5 *µ*g/ml). Plasmocin can be used at a concentration of 1 to 50 *µ*g/ml (e.g., 10 to 40 *µ*g/ml, or about 25 *µ*g/ml). Tylosin can be used at a concentration of 0.012 to 1.2 mg/ml (e.g., 0.06 to 0.6 mg/ml, or about 0.12 mg/ml).

Growth medium used for cell culture can be supplemented with antibiotics to prevent contamination of the cells by, for example, bacteria, fungus, yeast, and mycoplasma. Mycoplasma contamination is a frequent and particularly vexatious problem in tissue culture. In order to prevent or minimize mycoplasma contamination, an agent such as tylosin can be added to the culture growth medium. The medium can be further supplemented with one or more antibiotics/antimycotics (e.g., gentamicin, ciprofloxacine, alatrofloxacine, azithromycin, MRA, plasmocin, and tetracycline). Tylosin can be used at a concentration of 0.006 to 0.6 mg/ml (e.g., 0.01 to 0.1 mg/ml, or about 0.06 mg/ml). Gentamicin can be used at a concentration of 0.01 to 0.1 mg/ml (e.g., 0.03 to 0.08 mg/ml, or about 0.05 mg/ml). Ciprofloxacine can be used at a concentration of 0.002 to 0.05 mg/ml (e.g., 0.005 to 0.03 mg/ ml, or about 0.01 mg/ml). Alatrofloxacine can be used at a concentration of 0.2 to 5.0 *µ*g/ml (e.g., 0.5 to 3.0 *µ*g/ml, or about 1.0 *µ*g/ml). Azithromycin can be used at a concentration of 0.002 to 0.05 mg/ml (e.g., 0.005 to 0.03 mg/ml, or about 0.01 mg/ml). MRA can be used at a concentration of 0.1 to 1.5 *µ*g/ml (e.g., 0.2 to 1.0*µ*g/ml, or about 0.75 *µ*g/ml). Plasmocin can be used at a concentration of 1 to 50 *µ*g/ml (e.g., 10 to 40 *µ*g/ml, or about 25 *µ*g/ml). Tetracycline can be used at a concentration of 0.004 to 0.1 mg/ml (e.g., 0.008 to 0.05 mg/ml, or about 0.02 mg/ml). The antibiotics can be present for the whole period of the culture or for a portion of the culture period.

Mycoplasma contamination can be assayed by an agar culture method using a system such as, for example, mycoplasma agar plates that are available from bioMérieux (Marcy l'Etiole, France) or can be prepared in house, or by PCR. The American Type Culture Collection (ATCC, Manassas, VA) markets a PCR "Mycoplasma Detection Kit". Culture medium containing tylosin (0.06 mg/ml), gentamicin (0.1 mg/ml), ciprofloxacine (0.01 mg/ml), alatrofloxacine (1.0 *µ*g/ml), azithromycin (0.01 mg/ml), and tetracycline (0.02 mg/ml) is particularly useful for preventing mycoplasma contamination. Another agent that can be useful in preventing mycoplasma contamination is a derivative of 4-oxo-quinoline-3-carboxylic acid (OQCA), which is commercially available as, for example, "Mycoplasma Removal Agent" from ICN Pharmaceuticals, Inc. (Costa Mesa, CA). This agent typically is used at a concentration of 0.1 to 2.5 mg/ml (e.g., 0.2 to 2.0 mg/ml, or 0.5 mg/ml). The antibiotic mixture or other agents can be present in the fibroblast cultures for the first two weeks after initiation. After a suitable time in culture (e.g., two weeks), antibiotic containing medium typically is replaced with antibiotic-free medium. Once a sufficient number of cells are present in the culture, they can be tested for mycoplasmal, bacterial and fungal contamination. Only cells with no detectable contamination are useful in methods of the invention.

A pharmaceutically acceptable carrier can be added to the cells before they are administered to a subject. The phrase "pharmaceutically acceptable" refers to molecular entities and compositions that are not deleterious to cells, are physiologically tolerable, and typically do not produce an allergic or similar untoward reaction, such as gastric upset, dizziness and the like, when administered to a human. Such compositions include physiologically acceptable diluents of various buffer content (e.g., Tris-HCl, acetate, phosphate), pH and ionic strength.

Alternatively, if the cells are not to be administered immediately, they can be incubated on ice at about 4°C for up to 24-48 hours post-harvest. For such incubation, the cells can be suspended in a physiological solution that has an appropriate osmolarity and has been tested for pyrogen and endotoxin levels. Such a solution typically does not contain phenol red pH indicator, and any serum preferably is the subject's serum (i.e., autologous serum) rather than fetal bovine serum (FBS) or another xenogeneic serum (e.g., horse serum or goat serum). Cells can be suspended in, for example, Krebs-Ringer solution containing 5% dextrose, or in any other physiological solution. The cells can be aspirated and administered to a subject in the incubation medium. The volume of saline or incubation medium in which the cells are suspended typically is related to factors such as the number of cells to be injected and the extent of the damage due to tissue degeneration or defect.

The cells used herein also can be frozen in any medium suitable for preserving cell types such as UMC, fibroblasts, and keratinocytes (e.g., any commercially available freezing medium). A medium consisting of about 70% (v/v) growth medium, about 20% (v/v) FBS and about 10% (v/v) dimethylsulfoxide (DMSO) is particularly useful. The FBS can be replaced with, for example, Krebs Ringer containing 5% dextrose, and the DMSO also can be replaced with glycerol, for example. Thawed cells can be used to initiate secondary cultures for the preparation of additional suspensions for later use in the same subject, thus avoiding the inconvenience of obtaining a second specimen.

### 2. Compositions containing autologous UMC

The invention provides compositions containing autologous UMC. These compositions are useful for treating conditions that include, for example, dental defects (e.g., a defect of the oral mucosa, trauma to the oral mucosa, or periodontal disease), bone defects and bone grafting, and cosmetic defects of the skin (e.g., laugh lines, stretch marks, wrinkles, wound scars, acne scars, or subcutaneous atrophy). UMC can be harvested and enriched in vitro by initiation of cultures from biopsies taken from a subject (e.g., a human). As described herein, UMC can be obtained from, for example, a skin biopsy or a biopsy of adipose tissue or bone marrow. UMC isolated from dermal tissue are particularly useful because they can be readily obtained and expanded. As used herein, the term "UMC" refers to cells that are at a "stage" of differentiation prior to fully differentiated connective tissue cells such as, for example, fibroblasts. Because UMC cannot differentiate into every type of somatic cell, UMC are different from pluripotent stem cells. In addition to fibroblasts, UMC can differentiate into adipose tissue, cartilage, tendon, bone, or muscle cells.

To generate a composition of the present invention, a UMC culture can be initiated from, for example, a full thickness (e.g., 1-5 mm, or more than 5 mm if enough tissue is available) dermal biopsy specimen of the gums, scalp, post-auriculum, palate, or skin of a subject. The dermis is located just beneath the epidermis, and typically has a thickness that ranges from 0.5 to 3 mm. A dermal specimen can be obtained using, for example, a punch biopsy procedure. Skin biopsies can be taken from skin that is located, for example, behind the ear. UMC can be isolated by, for example, harvesting and initiation of cultures from a dermal (e.g., skin) biopsy (e.g., see Example 1). Floating (i.e., non-adherent) colonies of actively growing UMC can be observed floating in such a culture. Colonies can be harvested by aspiration and centrifugation of culture medium from the cell culture, and can be expanded by reseeding into fresh tissue culture medium. The floating colonies can be harvested when the adherent cells in the culture are confluent or non-confluent. Colonies of floating UMC also can be isolated from cultures of adipose tissue (e.g., fat harvested by liposuction or other surgical removal). The tissue can be cut into small pieces, membranous material can be removed, and the resulting tissue can be placed in culture under conditions that lead to active shedding of UMC from the adipose tissue. Examples of conditions that lead to active shedding include, but are not limited to, use of a culture medium containing decreased concentrations of foetal bovine serum (FBS) or culture medium containing acidic fibroblast growth factor (aFGF). For example, from about 0% to about 2.5% FBS may be present. For example, from about 2.5 to 10ng/ml aFGF may be present. Alternatively, the adipose tissue can be dissociated with about 0.1% to 1% collagenase after removal of membranes from the fat globules. Similarly, UMC cultures can be initiated from biopsies of bone marrow (see, e.g., Marko et al. (1995) Endocrinol. 136:4582-4588).

Any tissue culture technique that is suitable for the propagation of UMC from biopsy specimens can be used to expand the cells. Useful techniques for expanding cultured cells can be found in, for example, R.I. Freshney, Ed., Animal Cell Culture: A Practical Approach, (IRL Press, Oxford, England, 1986) and R.I. Freshney, Ed., Culture of Animal Cells: A Manual of Basic Techniques, (Alan R. Liss & Co., New York, 1987).

Cell culture medium can be any medium suitable for the growth of primary UMC cultures. Culture medium can contain antibiotics, antimycotics, and/or reagents that prevent the growth of mycoplasma, as described above. The presence of acidic fibroblast growth factor (aFGF), insulin-like growth factor-I (IGF-I), insulin, or any growth factor in the culture medium can prevent the UMC from differentiating into fibroblasts. The medium can be serum-free, or can be supplemented with human or non-human serum [e.g., autologous human serum, non-autologous human A/B serum, horse serum, or fetal bovine serum (FBS)] to promote growth of the cells. When included in the medium, serum typically is in an amount between about 0.1 % and about 20% v/v (e.g., between about 0.5% and about 19%, between about 1% and about 15%, between about 5% and about 12%, or about 10%). A particularly useful medium contains glucose DMEM that is supplemented with about 2 mM glutamine, about 10 mg/L sodium pyruvate, about 10% (v/v) FBS, and antibiotics (often called "complete medium"), wherein the concentration of glucose ranges from about 1,000 mg/L to about 4,500 mg/L. UMC also can be expanded in serum-free medium; in this way, the UMC are never exposed to xenogeneic or allogeneic serum proteins and do not require the extra culturing in serum-free medium that is carried out when the cells are expanded in medium that contains non-autologous serum.

Prior to administration, UMC can be incubated with an activating compound. As used herein, an "activating compound" is a compound that can stimulate cell proliferation or enhance cell longevity, (e.g., by stimulating expression of particular genes that lead to cell proliferation, or by activating DNA repair and prolonging cell life or preventing apoptosis). After UMC have differentiated into fibroblasts, activating compounds also can stimulate collagen production. Activating compounds also can be administered to a subject together with or separately from a composition containing UMC, and can enhance proliferation, longevity, or collagen production of cells in the area to which the UMC-containing composition is administered. When the cell composition and the activating compound are administered separately, the administrations can be simultaneous or sequential (in any order). Where sequential, the administrations can be minutes (e.g., 1, 2, 3,4, 5, 6, 7, 8, 9, 10, 15, 20, 30, 40, 50, or 55 minutes) apart, hours (e.g., 1, 2, 3, 4, 5, 6, 7, 8, 9, 10, 11, 12, 14, 18, 22, or 23 hours) apart, days (e.g., 1, 2, 3, 4, 5, or 6 days) apart, or weeks (e.g., 1, 2, 4, 5, 6, 7, 8, 9, 10, 12, 15, 20, 25, 30, 40, 50, or more weeks) apart. Alternatively, administration of one or more activating compounds in the absence of passaged UMC can be used to stimulate or differentiate UMC in vivo.

Other compounds that can be administered with UMC (and/or other cells such as fibroblasts) are enhancing compounds. As used herein, "enhancing compounds" are compounds that that do not necessarily act on the administered cells per se but, for example, either are structural components of the tissues to be repaired (e.g., any type of collagen) or act on host components (e.g., host cells or extracellular matrix components). Enhancing compounds thus can serve to enhance the reparative efficacy of the administered cells. Enhancing compounds can be administered to a subject together with or separately from a composition containing UMC and/or other cells such as fibroblasts. When the cell composition and the enhancing compound are administered separately, the administrations can be simultaneous or sequential (in any order). As described above, sequential administrations can be minutes (e.g., 1, 2, 3, 4, 5, 6, 7, 8, 9, 10, 15, 20, 30, 40, 50, or 55 minutes) apart, hours (e.g., 1, 2, 3, 4, 5, 6, 7, 8, 9, 10, 11, 12, 14, 18, 22, or 23 hours) apart, days (e.g., 1, 2, 3, 4, 5, or 6 days) apart, or weeks (e.g., 1, 2, 4, 5, 6, 7, 8, 9, 10, 12, 15, 20, 25, 30, 40, 50, or more weeks) apart. Alternatively, administration of one or more enhancing compounds in the complete absence of passaged UMC can be used to stimulate or differentiate UMC in vivo.

Activating compounds with which UMC may be admistered include, for example, ascorbic acid, ascorbyl palmitate, linoleic acid, C-MED 100® (Optigene-X LLC, Shrewsbury, NJ), quinic acid, quinic acid salts, quinic lactones, CoEnzyme Q-10, L-hydroxy acid, L-lipoic acid, niacin, dehydroepiandrosterone (DHEA), dimethylamino ethanol (DMAE), α-tocopherol, vitamin E, vitamin C, carotenoids, glycolic acid, carboxyalkyl esters, estriol, acetyl-L-carnitine, deprenyl, lycopene, nicotinamide adenine dinucleotide (NADH), cysteine, procysteine, pikamilon, vinpocetine, retinoic acid, antineoplastons, and other stimulatory additives such as growth factors [e.g., human growth hormone, fibroblast growth factor (FGF), vascular endothelial growth factor (VEGF), IGF-I, insulin, long form R3 IGF-I, and transforming growth factor-β (TGF- β)]. As indicated above, these activating compounds can also be administered to a subject together with, or separately from, UMC. Compounds useful as enhancing compounds include aminocaproic acid, estriol, and acetylcholinesterase, for example. Some of these compounds are antioxidants (e.g., L-lipoic acid, C-MED 100® , and CoEnzyme Q-10), others are thought to affect DNA repair (e.g., vitamin E, carboxyalkyl esters, and carotenoids), and others can induce the development of bone cells (e.g., calcium triphosphate and calcium monophosphate). Other useful activators include transcription factors, histone acetyl transferases, methyl binding proteins, acetylcholine precursors, cell membrane protectors, and differentiation factors.

It is understood that where activating or enhancing compounds of interest are proteins, cells to be administered to a subject (e.g., UMC or fibroblasts) can be stably or transiently transfected, transduced, transformed, or infected with one or more (e.g., one, two, three, four, five, six, or more) expression vectors (e.g., plasmids or viral vectors such as vaccinia, adenoviral, or retroviral vectors), each containing one or more (e.g., one, two, three, four, five, six, or more) nucleic acid sequences encoding one or more (e.g., one, two, three, four, five, six, or more) activating and/or enhancing compounds (e.g., any type of collagen (such as type I, type II, or type III collagen), a transcription factor, telomerase, or a factor that affects DNA methylation).

Cells (e.g., UMC) also can be activated by exposure to low energy laser light prior to administration to a subject. For example, cells can be irradiated with a helium-neon laser or a gallium-arsenide laser, which can increase collagen production by fibroblasts (see, e.g., Halcin and Uitto, "Biologic Effects of Low-Energy Lasers," in Lasers in Cutaneous and Aesthetic Surgery, 1 st edition (1997), Arndt and Dover, eds., Lippincott Williams & Wilkins (Philadelphia, PA), pp. 303-328). Treatment with a laser also may affect cell proliferation, immune modulation, and extracellular matrix production in wound healing. Cells can be irradiated one or more times (e.g., two, three, four, five, or more than five times) over any suitable period of time (e.g., several hours to several weeks) with a low-level laser prior to administration of the cells to a subject.

The invention also provides methods for making compositions containing autologous, passaged UMC. The methods can involve providing a biopsy of UMC-containing tissue from a subject, separating autologous UMC from the biopsy, culturing the UMC under conditions that result in cells that are substantially free of culture medium serum-derived proteins. Where UMC are prepared as non-adherent cell colonies in cultures of adherent fibroblasts derived from, for example, a skin biopsy, they can be passaged or collected by removal of the non-adherent cell-containing medium from the cultures and centrifugation. Where the UMC are to be passaged, they are resuspended in fresh culture medium and dispensed into an appropriate tissue culture vessel, e.g., a tissue culture flask. Where the UMC are to be administered to a subject they are resuspended in an appropriate solution (e.g., normal saline, Krebs Ringer with 5% dextrose, tissue culture media that is phenol red free, or any other solution that is isotonic and suitable for injection into a subject such as a mammal or a human). Any other suitable method also can be used to isolate and culture UMC, including methods disclosed in U.S. Patent No. 5,858,390, which is incorporated herein by reference in its entirety.

### 3. Compositions containing autologous fibroblasts and UMC

In addition to UMC, compositions of the invention can contain autologous, passaged fibroblasts that are substantially free of immunogenic proteins (e.g., culture medium serum-derived proteins). Compositions can contain any fibroblast that can be expanded in culture. Fibroblasts isolated from dermal tissue are particularly useful because they can be readily obtained and expanded. However, autologous fibroblasts can be obtained from any tissue that contains fibroblasts, e.g., a biopsy of the gums, palate, or skin of the subject. In addition, fibroblasts can be obtained from, without limitation, fascia, lamina propria, the bulbar area of hair follicles, bone marrow, or any source of connective tissue. In addition, fibroblasts can be derived from UMC, e.g., by cell culture. Any suitable method for culturing and differentiating UMC can be used, e.g., as described herein. UMC can be induced to differentiate into fibroblasts by, for example, culturing in the absence of acidic fibroblast growth factor or any other appropriate differentiation-inhibiting factor known in the art.

If desired, sterile microscopic dissection can be used to separate dermal tissue in a biopsy from keratinized tissue-containing epidermis and from adipocyte-containing subcutaneous tissue. The biopsy specimen then can be separated into small pieces using, for example, a scalpel or scissors to finely mince the tissue. In some embodiments, the small pieces of tissue are digested with a protease (e.g., collagenase, trypsin, chymotrypsin, papain, or chymopapain). Digestion with 200-1000 U/ml of collagenase type II for 10 minutes to 24 hours is particularly useful, although any type of collagenase can be used (e.g., 0.05% to 0.1% collagenase type IV can be particularly useful for digestion of fat tissue). If enzymatic digestion is used, cells can be collected by centrifugation and plated in tissue culture flasks.

If the tissue is not subjected to enzymatic digestion, minced tissue pieces can be individually placed onto the dry surface of a tissue culture flask and allowed to attach for between about 2 and about 10 minutes. A small amount of medium can be slowly added so as not to displace the attached tissue fragments. In the case of digested cells, the cells can be washed with culture medium to remove residual enzyme, suspended in fresh medium, and placed in one or more flasks. After about 48-72 hours of incubation, flasks can be fed with additional medium. When a T-25 flask is used to start the culture, the initial amount of medium typically is about 1.5-2.0 ml. The establishment of a cell line from the biopsy specimen can take between about 2 and 3 weeks, at which time the cells can be removed from the initial culture vessel for expansion.

During the early stages of the culture, it is desirable that the tissue fragments remain attached to the culture vessel bottom. Fragments that detach can be reimplanted into new vessels. The fibroblasts can be stimulated to grow by a brief exposure to EDTA-trypsin, according to standard techniques. Such exposure to trypsin is too brief to release the fibroblasts from their attachment to the culture vessel wall. Immediately after the cultures become established and are approaching confluence, samples of the fibroblasts can be processed for frozen storage in, for example, liquid nitrogen. Any suitable method for freezing cells can be used, including any of the numerous methods that are known in the art for successfully freezing cells for later use. See, e.g., the freezing methods disclosed above. The freezing and storage of early rather than late passage fibroblasts is preferred because the number of passages in cell culture of normal human fibroblasts is limited.

Any tissue culture technique that is suitable for the propagation of dermal fibroblasts from biopsy specimens can be used to expand the cells. Cell culture medium can be any medium suited for the growth of primary fibroblast cultures. The medium can be supplemented with human or non-human serum [e.g., autologous human serum, non-autologous human A/B serum, horse serum, or fetal bovine serum (FBS)] as described above. Fibroblasts also can be expanded in serum-free medium; in this way, the fibroblasts are never exposed to xenogeneic or allogeneic serum proteins and do not require the extra culturing in serum-free medium that is carried out when the fibroblasts are expanded in medium that contains non-autologous serum.

Autologous fibroblasts can be passaged into new flasks by trypsinization. For expansion, individual flasks can be split at a ratio of, for example, 1:3 to 1:5. Triple bottom, T-150 flasks having a total culture area of 450 cm² are suitable for expanding fibroblasts. A triple bottom T-150 flask can be seeded with, for example, about 1x10⁶ to about 3x10⁶ cells, or about 8x10³ cells per square centimeter, depending on the size of the cells. Such a flask typically has a capacity to yield about 6x10⁶ to about 1x10⁷ cells. When the capacity of the flask is reached, which can require about 5-7 days of culture, the growth medium can be replaced by serum-free medium. The cells typically are incubated between about 30°C and about 37.5°C for at least 4 hours (e.g., overnight or about 18 hours). Incubation of the cells in serum-free medium can substantially remove proteins derived from the non-autologous serum (e.g., FBS) added to the culture medium, which if present in a composition injected into a subject, could elicit an undesirable immune response. Serum-free medium can contain, for example, glucose DMEM supplemented with about 2 mM glutamine, with or without about 110 mg/L sodium pyruvate, wherein the concentration of glucose can range from approximately 1,000 mg/L to about 4,500 mg/L. A glucose concentration of approximately 4,500 mg/L is particularly useful. The serum-free medium also can contain one or more antibiotics such as those described above.

At the end of the incubation in serum-free medium, the cells can be removed from the tissue culture flasks using, for example, trypsin-EDTA. Prior to administration to a subject, fibroblasts typically are washed 2 to 4 times in medium that is serum-free and phenol red-free, or in saline. Cells can be washed by centrifugation and resuspension, and then suspended for injection in an equal volume of injectable isotonic solution that has an appropriate physiological osmolarity and is substantially free of pyrogens and foreign proteins. Isotonic saline is a particularly useful isotonic solution. Five triple bottom T-150 flasks, grown to capacity, can yield about 3.5x10⁷ to about 7x10⁷ cells, which are sufficient to make up about 1.2 ml to about 1.4 ml of suspension. A pharmaceutically acceptable carrier can then be added to the passaged autologous fibroblasts to form a pharmaceutical composition.

As for UMC, fibroblasts can be incubated with an activating compound before administration to a subject (see above). Suitable activating compounds include those described above, for example. Incubation with such compounds can stimulate the fibroblasts and enhance their collagen production. One or more activating compounds also can be administered to a subject along with a composition containing autologous, passaged fibroblasts. Alternatively, administration of an activating compound in the absence of passaged fibroblasts can be used to stimulate fibroblasts in vivo.

Moreover, the same enhancing compounds described above for in vivo use with UMC also can be used with compositions containing UMC and fibroblasts, or even with fibroblasts alone.

As above, fibroblasts can be activated by irradiation with a low-energy laser.

Any other suitable method also can be used to prepare compositions containing autologous, passaged fibroblasts. See, e.g., U.S. Patent Nos. 5,858,390; 5,665,372; 5,660,850; 5,591,444; and 6,342,710, as well as International Application No. WO 99/60951, all of which are incorporated by reference in their entirety.

The invention provides methods for making compositions that contain autologous, passaged UMC and autologous, passaged fibroblasts. The methods can involve (1) providing a biopsy ofUMC-containing tissue from a subject, separating UMC from the biopsy, and culturing the UMC for a number of passages suitable to obtain the desired quantity of cells, under conditions that result in UMC that are substantially free of culture medium serum-derived proteins; (2) providing a biopsy of fibroblast-containing tissue from the subject, separating fibroblasts from the biopsy, culturing the fibroblasts through a number of passages suitable to obtain the desired quantity of cells, under conditions that result in fibroblasts that are substantially free of culture medium serum-derived proteins, and exposing the fibroblasts to conditions (e.g., trypsin) that result in suspension of the fibroblasts; and (3) combining the UMC with the fibroblasts. It is noted that UMC and fibroblasts can be obtained from a single biopsy specimen and, furthermore, can be co-cultured. The final combining step thus may not be necessary.

### 4. Compositions containing autologous keratinocytes

Compositions of the invention can contain passaged keratinocytes. The keratinocytes can be autologous or non-autologous (e.g., from another subject or from a cell line), although autologous keratinocytes are particularly useful. Autologous keratinocytes can be isolated from, for example, a biopsy of skin, gums, buccal tissue, esophageal tissue, exocervical tissue, hair follicles, or conjunctival tissue. Autologous keratinocytes typically are obtained from sites that are easily accessible yet not highly visible from a cosmetic standpoint. Non-autologous keratinocytes can be from keratinocyte cell lines. Such cells are commercially available from, for example, the ATCC. The keratinocytes used herein typically are in suspension and can be administered to a subject by, for example, injection.

The invention provides methods for making a composition for repairing or augmenting a tissue defect such as a burn wound in a subject. During a typical healing process, keratinocytes grow from the outer edge of a wound toward the center of the wound, which often results in scarring. Compositions containing suspensions of keratinocytes can be administered (e.g., injected) to burn wounds to facilitate healing while reducing scarring. The keratinocyte suspensions are substantially free of immunogenic proteins (e.g., culture medium serum-derived proteins).

Keratinocytes can be harvested from, for example, a skin biopsy. Full thickness or split thickness human skin can be used. The skin can be obtained from any part of the body, including foreskin. If full thickness skin is used, the dermis can be trimmed down as far as possible. The skin can be washed one or more times in medium (e.g., MEM) containing antibiotics and/or antimycotics (e.g., penicillin, streptomycin, and Fungizone®), or in the antibiotic-containing medium described above. The tissue then can be cut into small (e.g., 4 mm²) pieces and incubated in trypsin (e.g., 0.25% trypsin in phosphate buffered saline, without Ca⁺⁺ or Mg⁺⁺) to separate the dermis from the epidermis [Eisinger (1985) Method in Skin Research, Ed. Skerrow, pp. 193]. Trypsin treatment can proceed at a suitable temperature (e.g., 4°C) for a suitable length of time (e.g., 2 to 18 hours). After trypsinization, the epidermis can be carefully separated from the dermis using fine forceps. The dermis can either be discarded or used for growth of fibroblasts, and the epidermis can be floated in a solution containing, for example, 8 g/L M NaCl, 0.4 g/L KCl, 1 g/L dextrose, 0.58 g/L NaHCO₃, 0.5 g/L trypsin, and 0.2 g/L versene (disodium salt).

When all epidermis is collected, the epidermal cells can be separated from each other by mechanical teasing with forceps. The cells eluted into the supernatant can be transferred into a new tissue culture dish and further separated by, for example, vigorous pipetting with a Pasteur pipette in order to obtain a suspension of single cells that can be observed under a microscope. Cells then can be collected into sterile fetal bovine serum (FBS) and filtered through, for example, sterile gauze mesh to remove any remaining clumps or pieces of tissue. Fresh medium can be added to the remaining epidermal tissue, and the procedure can be repeated until all tissue is completely disaggregated. The cells collected in FBS can be pelleted by centrifugation, resuspended in a keratinocyte growth medium, and seeded at about 5 x 10⁵ to 8 x 10⁵ cells/ml in plastic tissue culture vessels. The cells typically are cultured in the presence of 5% CO₂ and 95% air, at 80% humidity and 35°C to 36°C. Keratinocytes can be ready for harvest after 12 to 14 days, and can be used for subculture or for administration to a subject.

Replicating epidermal keratinocyte cultures can be maintained on fibroblast feeder layers (e.g., layers of 3T3 cells), or can be cultured in the presence of animal serum (e.g., FBS), both for establishing primary cultures and for subcultures. Under these conditions, keratinocytes can be subcultured until they achieve 20 to 50 population doublings. Cells can be cultured in, for example, minimal essential medium (MEM) containing 10% FBS, 100 U/ml penicillin, 100 g/ml streptomycin, 0.6 *µ*g/ml Fungizone®, 0.5 *µ*g/ml hydrocortisone, 200 mM L-glutamine, and 10 mM MEM-non-essential amino acids. Rather than penicillin, streptomycin and Fungizone® , the antibiotic mixtures described above for UMC and fibroblasts cultures also can be used. In vitro systems that do not require serum or feeder layers to support clonal growth, serial propagation, and differentiation of epidermal keratinocytes also have been developed [Peehl and Ham (1980) In Vitro 16:516-525; and Tsao et al. (1982) J. Cell. Physiol. 110:219-229]. Such systems can include the use of conditioned medium from fibroblast cultures.

Keratinocyte culture conditions can be modified to enhance cell growth. For example, increased hydrocortisone concentrations (e.g., 10 *µ*g/ml), putrescine (e.g., 10⁻⁵ M), vitamin B12 (e.g., 10⁻⁵ M), or J-estradiol (e.g., 3.7 x 10⁻⁶ M) can be used to enhance keratinocyte growth in unconditioned medium [see, e.g., Peehl and Ham supra]. Keratinocyte growth medium also can contain components such as epidermal growth factor and/or insulin. Calcium concentrations also can be adjusted to affect the rate of keratinocyte proliferation [Hennings et al. (1980) Cell 19:245-265]. Culturing epidermal cells in medium containing 0.05 to 0.1 mM Ca⁺⁺, for example, can increase the rate of proliferation, while growth under conditions normally found in culture media (e.g., 1.2 mM Ca⁺⁺) can result in terminal differentiation of the cells. Any other suitable method for isolating and culturing keratinocytes can be used to produce the compositions provided herein. These include methods disclosed in, for example, U.S. Patent Nos. 4,254,226; 5,282,859; and 6,029,760, which are incorporated herein by reference in their entirety.

The invention provides methods for making compositions that contain keratinocytes. The methods can involve providing a biopsy of keratinocyte-containing tissue, separating keratinocytes from the tissue biopsy, culturing the keratinocytes through a number of passages suitable to expand the cells to a desired quantity, under culture conditions that result in cells that are substantially free of culture medium serum-derived proteins, and exposing the cells to conditions (e.g., trypsin) that result in suspension of the cells.

Compositions containing keratinocytes can also contain fibroblasts (e.g., autologous, passaged fibroblasts) and/or UMC (e.g., autologous, passaged UMC). Fibroblasts can be isolated and suspensions can be prepared as described herein (see subsection 3, above). The methods provided herein involve obtaining one or more tissue biopsies, preparing suspensions of autologous, passaged keratinocytes and autologous, passaged fibroblasts, and combining the keratinocytes with the fibroblasts to generate a composition that can be used to treat a condition such as a burn wound. It is noted that keratinocytes and fibroblasts can be obtained and prepared from a single biopsy (e.g., a full-thickness skin biopsy). Any suitable ratio of fibroblasts to keratinocytes can be used, although a ratio of about 3:1 (fibroblasts to keratinocytes) is particularly useful.

### 5. Compositions containing biodegradable acellular matrix components

Compositions of the invention that contain autologous, passaged cells (e.g., UMC with or without fibroblasts) also can include biodegradable acellular matrix components. An acellular matrix component generally fulfils a structural role. For example, it may fill in a defect, hole, space or cavity in tissue and provide an environment in which injected cells can adhere to the matrix or surrounding tissue and grow and produce structural and other factors (e.g chemotactic factors) resulting from the growth of new tissue. In many instances, the gap-filling function of the matrix is temporary and only lasts until the implanted and/or host cells migrate into the area and form new tissue. Preferably the acellular matrix is biodegradable. The matrix is preferably a solid or semi-solid substance that is insoluble under physiological conditions. In addition, biodegradable acellular matrix components also can be included in any of the compositions containing passaged keratinocytes. Such compositions are suitable for injection or implantation into a subject to repair tissue that has degenerated. The term "biodegradable" as used herein denotes a composition that is not biologically harmful and can be chemically degraded or decomposed by natural effectors (e.g., weather, soil bacteria, plants, animals). Examples of matrices that can be used in the present invention include, without limitation, acellular matrices containing autologous and non-autologous proteins, and acellular matrices containing biodegradable polymers.

Any of a number of biodegradable acellular matrices containing non-autologous proteins can be used in the compositions provided herein. Examples of biodegradable acellular matrices include matrices containing any type of collagen (e.g., bovine, porcine, human, or bio-engineered collagen), or any type of collagen with glycosaminoglycans (GAG) cross-linked with, for example, glutaraldehyde. Matrices containing collagen include, without limitation, absorbable collagen sponges, collagen membranes, and bone spongiosa. Useful types of collagen include, for example, bovine collagen (e.g., ZYDERM® and ZYPLAST® , commercially available from McGhan Medical Corporation, Santa Barbara, CA), porcine collagen, human cadaver collagen (e.g., FASCIAN^{TM} (Fascia Biosystems, LLC, Beverly Hills, CA), CYMETRA^{TM} (LifeCell Corp., Branchburg, NJ), or DERMALOGEN^{TM} (formerly produced by the Collagenesis Corp.), bioengineered collagen (e.g., FORTAPERM^{TM}, available from Organogenesis, Inc., Canton, MA), and autologous human collagen (AUTOLOGEN® , see below). FASCIAN^{TM} can be particularly useful. This product is available in five different particle sizes, any of which can be used in compositions and methods described herein. Particles that are 0.25 mm in size can be particularly useful.

Absorbable collagen sponges can be purchased from, for example, Sulzer Calcitek, Inc. (Carlsbad, CA). These collagen sponge dressings, sold under the names COLLATAPE® , COLLACOTE® , and COLLAPLUG® , are made from cross-linked collagen extracted from bovine deep flexor (Achilles) tendon, and GAG. These products are soft, pliable, nonfriable, and non-pyrogenic. Greater than 90% of a collagen sponge typically consists of open pores.

Biodegradable acellular matrices can contain collagen (e.g., bovine or porcine collagen type I) formed into, for example, a thin membrane. One such membrane is manufactured by Sulzer Calcitek and is marketed as BIOMEND^{TM}. Another such membranous matrix is marketed as BIO-GIDE® by Geistlich Söhne AG (Wolhusen, Switzerland), and is made of porcine type I and type III collagen. BIO-GIDE® has a bilayer structure, with one surface that is porous and allows the ingrowth of cells, and a second surface that is dense and prevents the ingrowth of fibrous tissue.

Other suitable matrices containing collagen include COLLAGRAFT® , manufactured by NeuCell, Inc. (Campbell, CA), and OSTEOSET® calcium sulfate alpha hemi-hydrate pellets sold by Wright Medical Technology (Arlington, TN).

Biodegradable acellular matrices also can be made from bone spongiosa formed into granules or blocks. This material consists of animal (e.g., human, non-human primate, bovine, sheep, pig, or goat) bone from which substantially all organic material (e.g., proteins, lipids, nucleic acids, carbohydrates, and small organic molecules such as vitamins and non-protein hormones) has been removed. This type of matrix is referred to herein as an "anorganic matrix". One such matrix, which is marketed as BIO-OSS® spongiosa granules and BIO-OSS® blocks, is manufactured by Geistlich Söhne AG. This company also manufactures a block-type matrix (BIO-OSS® collagen) that contains anorganic bone and additionally contains approximately 10% collagen fibers by weight.

Other useful biodegradable acellular matrices can contain gelatin, cat gut, demineralized bone, anorganic bone, coral, or hydroxyapatite, or mixtures of these substances. A matrix made from demineralized human bone, for example, is formed into small blocks and marketed as DYNAGRAFT® by GenSci Regeneration Laboratories, Inc. (Toronto, Ontario), TUTOPLAST® by Tutogen Medical, Inc. (Clifton, NJ), or GRAFTON® Demineralized Bone Matrix by Osteotech, Inc. (Eatontown, NJ). Demineralized bone can be combined with, for example, collagen to produce a matrix in the form of a sponge, block, or membrane. Biodegradable matrices can contain glycosaminoglycans such as mucopolysaccharide or hyaluronic acid. In addition, synthetic polymers made from one or more monomers can be used to make biodegradable acellular matrices that are useful herein. Such synthetic polymers include, for example poly(glycolic acid), poly(lactic acid), and poly(glycolic acid)-poly(lactic acid). Synthetic polymers also can be combined with any of the above-mentioned substances to form matrices. Different polymers forming a single matrix can be in separate compartments or layers. For example, W. L. Gore & Associates, Inc. (Flagstaff, AZ) manufactures a porous biodegradable acellular matrix (GORE RESOLUT XT Regenerative Material). This matrix is composed of a synthetic bioabsorbable glycolide and trimethylene carbonate copolymer fiber into which cells can migrate, attached to an occlusive membrane that is composed of a synthetic bioabsorbable glycolide and lactide copolymer that does not permit ingrowth of cells. Other examples of suitable biodegradable matrices can be found in United States Patent No. 5,885,829, for example.

After a biodegradable acellular matrix has been selected, a concentrated suspension of cells (e.g., autologous passaged UMC with or without autologous, passaged fibroblasts) can be evenly distributed on the surface of the matrix. A concentrated suspension typically is used in order to avoid exceeding the capacity of the matrix to absorb the liquid suspension. For example, a cell suspension applied to a GORE RESOLUT XT matrix generally can have a volume between about 94 *µ*l and about 125 *µ*l and contain between about 2.0x10⁶ cells and about 4.0x10⁶ cells per square centimeter of matrix. Cells can be allowed to attach to the matrix without further addition of media. Incubation of the cells with the matrix can be at, for example, about 37°C for about 1-2 hours. Cells typically are attached to and evenly distributed throughout the matrix material after about sixty minutes of incubation. At this time, the culture vessels containing the cell-loaded matrices can be supplemented with additional growth medium, and cells can be cultured in the matrix for about 3 to 4 days. Because the cells are added to the matrix at high density so as to substantially fill the space within the matrix, little or no proliferation occurs during the 3-4 day culture period. Indeed, significant cell proliferation typically is undesirable during this period because dividing fibroblasts can secrete enzymes (e.g., collagenase) that can degrade or partially degrade the matrices.

The matrix with the cells typically is washed (e.g., at least 3 washes of 10 minutes each) with, for example, saline or medium that is free of serum and phenol red, in order to substantially remove immunogenic proteins (e.g., culture medium serum-derived proteins if medium containing non-autologous serum was used for the matrix seeding step) that could elicit an immune response when administered to a subject. Fresh PBS can be used for each wash. The matrix then can be incubated (e.g., 2 hour-long incubations) in fresh PBS or serum-free culture medium prior to use. After incubation, the matrix containing autologous, passaged UMC with or without passaged fibroblasts can be placed at the area of tissue degeneration or defect.

For collagen sponge matrices (e.g. COLLACOTE® ), approximately 1.5x10⁷ to 2.0x10⁷ cells in approximately 1.5 ml of growth medium can be seeded onto a 2 cm by 4 cm thin (approximately 2.5 to 3.0 mm in thickness) sponge. The sponge then can be incubated at 37°C for about 1-2 hours without further addition of medium to allow substantially all cells to adhere to the matrix material. After cell adherence, additional growth medium can be added to the matrix and cell composition, which then can be incubated at 37°C for 3-4 days with a daily change of medium. If medium containing non-autologous serum was used for the cell seeding step, the composition can be removed from growth medium containing such serum and washed repeatedly (e.g., 3 times or more) with PBS. After each addition of PBS, the matrix can be incubated for 10-20 minutes prior to discarding the PBS. After the final wash, the composition can either be administered immediately to a subject, or can be transferred to a shipping vial containing a physiological solution (e.g., Kreb's Ringer solution) and incubated at about 4°C for up to about 24-48 hours.

For a membranous matrix (e.g. BIOMEND^{TM}), approximately 3x10⁶ to 8x10⁶ cells (e.g., UMC or UMC with fibroblasts) in about 100 *µ*l of growth medium can be seeded onto a 15 mm x 20 mm thin (approximately 0.5 to 1.0 mm in thickness) membrane. The membrane can be incubated at 37°C for about 30-60 minutes without further addition of medium to allow substantially all of the cells to adhere to the matrix material. After cell adherence, additional growth medium can be added to the matrix and cell composition, which then can be incubated at 37°C for 2-3 days with a daily change of medium. The cells typically are added to the matrix at high density (see above) so as to substantially fill the space within the matrix available for cells. Washing of the composition and either immediate use or incubation can be as described above for the sponge matrices.

In the case of a block matrix such as the above described anorganic matrix (e.g., the BIO-OSS® block) or a demineralized bone matrix (e.g., the DYNAGRAFT^{TM} matrix), approximately 1.2x10⁷ to 2.0x10⁷ cells in approximately 100 to 150 *µ*l of growth medium can be seeded into a 1 cm x 1 cm x 2 cm cubic block of matrix material. Cells typically are seeded slowly onto one face of the block face. Once the medium and cells have been absorbed into the block, another face of the block can be seeded in a similar fashion. The procedure can be repeated until all faces of the block have been seeded and the block is fully saturated with medium. Care should be taken to avoid adding excess medium and thereby causing leakage of medium and cells from the block. The composition then can be incubated at 37°C for about 60-120 minutes without further addition of medium to allow substantially all the cells to adhere to the matrix material. After cell adherence, additional growth medium can be added to the matrix and cell composition, which then can be incubated at 37°C for 2-3 days with a daily change of medium. The cells typically are added to the matrix at high density (see above) so as to substantially fill the space within the matrix available for cells with the same result described above. Washing of the composition and either immediate use or incubation are as described above for the sponge matrices.

Compositions containing autologous, passaged cells and a small particle biodegradable matrix (e.g., FASCIAN^{TM}, CYMETRA^{TM}, or DERMALOGEN^{TM}) can be prepared by mixing the components by, for example, passing them back and forth between two syringes that are connected via a luer lock. FASCIAN^{TM}, for example, is typically available in syringes (e.g., 3 cc syringes) at 80 mg/syringe. FASCIAN^{TM} particles can be washed directly in the syringe prior to use by taking up a small volume (e.g., 1.5 ml) of a wash buffer (e.g., isotonic saline or Kreb's Ringers solution containing dextrose) into the syringe, connecting the first syringe to a second syringe via a luer lock, and passing the particles and wash solution back and forth between the two syringes several times. To separate the particles from the wash solution, the mixture can be transferred to a sterile tube and the FASCIAN^{TM} particles allowed to settle. The solution can be removed (e.g., decanted or aspirated), and the washing process can be repeated as desired by taking up the particles into a fresh syringe (e.g., through an 18 gauge or 20 gauge needle).

When the particles are suitably washed, they can be mixed with cells (e.g., UMC and, optionally, fibroblasts) using the same procedure as for washing. Cells (e.g., 1x10⁷ to 3x10⁷ cells) can be suspended in solution (e.g., 1.5 ml of Kreb's Ringers solution with 5% dextrose) and taken up into a syringe. The syringe containing the cells can be connected to a syringe containing the filler particles via a luer lock, and the two components can be mixed by passing them back and forth between the syringes. The mixture then can be transferred to a T-25 tissue culture flask or to a tissue culture dish or a tube so that the cells can attach to the filler particles. Alternatively, the mixture can remain in the syringes while attachment occurs, although this may be more detrimental to the cells. The mixture can be incubated over night and then transferred to a container (e.g., a vial or a tube) for delivery to a clinician, or transferred to a syringe for administration to a subject. A container to be delivered to a clinician can be kept on ice during delivery. When such small particle acellular biodegradable matrices are used, a suspension of the cell-containing particles can optionally be injected rather than implanted into an area of tissue degeneration or defect.

When two cell types (e.g., UMC and fibroblasts) are included in a composition containing a biodegradeable acellular matrix, the cells can be mixed together prior to seeding into the matrices. Alternatively, they can be seeded separately into the matrices. When one cell type (e.g., fibroblasts) is seeded before or after another cell type (e.g., UMC), the second seeding can be performed immediately after the first seeding or after the cells of the first seeding have substantially adhered to the matrix material.

The invention also provides methods for making compositions that contain both cells (e.g., autologous, passaged UMC) and matrix components. These methods typically involve providing a suspension of autologous, passaged cells that are substantially free of immunogenic proteins (e.g., culture medium serum-derived proteins), providing a biodegradable acellular matrix, incubating the biodegradable acellular matrix with the cell suspension such that the cells integrate on and within the matrix, thus forming a composition for repairing or augmenting tissue. These methods also can include adding a second suspension of cells (e.g., autologous, passaged fibroblasts) to the matrix, either together or separately from the first cell suspension.

### 6. Compositions containing filler materials

Compositions of the invention can contain cells (e.g., autologous, passaged UMC) together with one or more biodegradable acellular injectable filler materials (i.e., bulking agents). The compositions are suitable for injection into a subject in order to repair tissue that has degenerated. A filler material generally fulfils a structural function. For example, it may fill in a defect, hole, space or cavity in tissue and provide an environment in which injected cells can adhere to the surrounding tissue and grow and produce structural and other factors (e.g chemotactic factors) resulting from the growth of new tissue. In many instances, the gap-filling function of the filler is temporary and only lasts until the implanted and/or host cells migrate into the area and form new tissue. Preferably the filler is biodegradable. Fillers are typically provided and used as a viscous solution or suspension. Fillers can be combined with one or more cell types (e.g., UMC and fibroblasts). Cells typically are combined with a filler in a ratio of approximately 1:1 by volume, although any suitable ratio can be used.

Numerous types of biodegradable, acellular injectable fillers can be added to compositions of the invention. A filler can consist of autologous proteins, including any type of collagen obtained from a subject. An example of such a filler is Autologen®, formerly produced by Collagenesis Corp. (Beverly, MA). Autologen® is a dispersion of autologous dermal collagen fibers from a subject, and therefore does not elicit even a minimal immune response when readministered to the subject with cells such as UMC and, optionally, fibroblasts. In order to obtain Autologen® , a specimen of tissue (e.g., dermis, placenta, or umbilical cord) is obtained from a subject and forwarded to Collagenesis Corp., where it is processed into a collagen-rich dispersion. Approximately one and a half square inches of dermal tissue can yield one cubic centimeter (cc) of Autologen® . The concentration of Autologen® can be adjusted depending upon the amount required to correct defects or augment tissue within the subject. The concentration of Autologen® in the dispersion can be, for example, at least about 25 mg/L (e.g., at least about 30 mg/L, at least about 40 mg/L, at least about 50 mg/L, or at least about 100 mg/L).

An acellular injectable filler material also can contain non-autologous proteins, including any type of collagen. Numerous collagen products are commercially available and can be used in compositions of the invention. Human collagen products also are commercially available. Examples of commercially available collagen include, without limitation, bovine collagen, e.g., reconstituted bovine collagen products such as Zyderm® and Zyplast® , which contain reconstituted bovine collagen fibers that are cross-linked with glutaraldehyde and suspended in phosphate buffered physiological saline with 0.3% lidocaine. These products are produced by McGhan Medical Corporation of Santa Barbara, CA. Porcine collagen products also are commercially available. Collagens useful in the invention can be isolated from tissues of appropriate species, or they can be made as recombinant proteins. Recombinant proteins can have amino acid sequences identical to those of the naturally occurring proteins, or they can have amino acid sequences containing substitutions, deletions, or insertions that improve the function of the proteins.

Other examples of useful filler materials include, but are not limited to, solubilized gelatin, polyglycolic acid (e.g., solubilized polyglycolic acid or particles of polyglycolic acid), or cat gut sutures. A particular gelatin matrix implant, for example, is sold under the mark Fibril® . This filler contains equal volumes of (1) a mixture of porcine gelatin powder and o-aminocaproic acid dispersed in a 0.9 % (by volume) sodium chloride solution, and (2) an aliquot of plasma from the subject. Other substances useful as fillers include hyaluron, hyaluronic acid, restalyn, and parleane.

When two cell types (e.g., UMC and fibroblasts) are included in a composition containing a biodegradeable acellular filler, the cells can be mixed together prior to mixing with the filler. Alternatively, they can be sequentially mixed with the filler. When one cell type (e.g., fibroblasts) is mixed with a filler before or after another cell type (e.g., UMC), the second mixing can be performed immediately after the first mixing or after a suitable incubation time.

The invention also provides methods for making compositions that contain cells (e.g., autologous, passaged UMC with or without autologous, passaged fibroblasts) and biodegradable acellular fillers. These methods typically involve providing a suspension of cells (e.g., autologous, passaged UMC and, optionally, fibroblasts) that are substantially free of immunogenic proteins (e.g., culture medium serum-derived proteins), providing one or more biodegradable acellular filler materials, and combining the filler with the cell suspension. Alternatively, separate suspensions of different cell types can be combined with a filler.

### 7. Methods for using compositions containing UMC with or without fibroblasts

The cell compositions of the invention can be employed to treat defects of, for example, skin, bone, or soft tissue. The cell compositions can be used in place of atelocollagen solutions with the advantages set forth as above. Diseases, disorders or defects resulting in degeneration of tissue that can be treated with the present invention include defects of the oral mucosa, trauma (e.g., extraction of a tooth) to the oral mucosa or oral bones such as the maxillary or mandibular bones, periodontal disease, diabetes, cutaneous ulcers, and venous stasis. In addition, examples of periodontal disease that result in tissue degeneration include, but are not limited to, periodontal degeneration, gingivitis, or non-healing wounds of the palatal mucosa or gingival mucosa, or bone degeneration. Other defects that can be treated with this invention include skin defects, such as scars, wrinkles, laugh lines, stretch marks, depressed scars, cutaneous depressions of non-traumatic origin, acne scarring, or subcutaneous atrophy from acne, trauma, congenital malformation, or aging. Moreover, compositions of the invention can be used to treat defects such a hypoplasia of the lips, labial folds, or bone defects, e.g., defects of bones such as, for example, facial bones including orbits, mandibles, maxillae, zygomatic bones, crania, and nasal bones, as well as spinal and long bones.

As used herein, "prophylaxis" can mean complete prevention of the symptoms of a disease, a delay in onset of the symptoms of a disease, or a lessening in the severity of subsequently developed disease symptoms. "Prevention" should mean that symptoms of the disease (e.g., cancer) are essentially absent. As used herein, "therapy" can mean a complete abolishment of the symptoms of a disease or a decrease in the severity of the symptoms of the disease.

Compositions of the invention can be administered to a subject to treat tissue defects (e.g., the defects described above). Administration can be by any suitable method, e.g., injection, implantation, or grafting.

The treatment of fine superficial facial lines, one embodiment of the invention, can be accomplished as follows. The area to be treated is prepped with alcohol and stretched to give a taut surface. A syringe is filled with a cell suspension and fitted with a 30 gauge or larger (e.g., 22 gauge, 18 gauge, or 12 gauge) needle for injection. The needle is inserted into the skin site as superficially as possible; the orientation of the bevel is not critical. Radiologic guidance can be used. An intradermal injection is made by gentle pressure until a slight blanch is seen. Multiple serial injections can be made.

In other embodiments, the injectate can be placed in the obicularis musculature to treat hypoplasia of the lip, or into the subcutaneous tissue to treat deep subcutaneous defects.

In an alternative embodiment, extensive areas of acne scarring can be treated by dermal abrasion to the level of the middle or deep dermis. A fibroblast containing clot then can be fashioned so as to cover the abraded surface and applied so that the fibroblast-seeded side of the clot is juxtaposed to the abraded dermal surface. The applied clot then can be covered with a surgical dressing such as Xeroform3, Adaptic3 or any nonocclusive surgical dressing.

In most cases where UMC and fibroblasts are administered, they are administered together, either as a single composition or as separate compositions. However, it is understood that the UMC and fibroblasts can be prepared separately and administered separately. Where the two cell populations are administered separately, the administrations can be simultaneous or sequential (in any order). Where sequential, the administrations can be minutes (e.g., 1, 2, 3, 4, 5, 6, 7, 8, 9, 10, 15, 20, 30, 40, 50, or 55 minutes) apart, hours (e.g., 1, 2, 3, 4, 5, 6, 7, 8, 9, 10, 11, 12, 14, 18, 22, or 23 hours) apart, days (e.g., 1, 2, 3, 4, 5, or 6 days) apart, or weeks (e.g., 1, 2, 4, 5, 6, 7, 8, 9, 10, 12, 15, 20, 25, 30, 40, 50, or more weeks) apart.

### 8. Methods for using compositions containing keratinocytes and fibroblasts

Keratinocytes make up the predominant cell population of the epidermis, and play a major role in accomplishing the repair and regeneration of damaged epidermal tissue. The invention provides methods for enhancing the repair of a tissue defect (e.g., a burn wound) by injecting a cell suspension that contains keratinocytes with or without fibroblasts. As described above, wound healing often results in scarring due to the growth of keratinocytes from the outer edges toward the center of the wound. By injecting keratinocytes (e.g., autologous, passaged keratinocytes) into the center regions of a wound, healing can be facilitated and scarring can be reduced or avoided altogether. Compositions containing keratinocytes and fibroblasts and/or UMC are particularly useful for treating bums (e.g., small bums or residual, unhealed sections of larger bums), although compositions containing only one of these cell types also can be used. Burn wounds to be treated typically are approximately three to five centimeters in diameter, but can be of any size or dimension. This type of treatment can serve to "fill in" and rebuild dermis that has been damaged by a wound such as a burn.

### 9. Devices

The invention provides devices for repairing dermal defects in a subject. Such devices can include a hypodermic syringe with a syringe chamber, a piston disposed in the syringe chamber, and an orifice communicating with the syringe chamber. The syringe chamber can contain a suspension of cells, e.g., a suspension of autologous, passaged UMC and autologous, passaged fibroblasts or any other cell composition or cell mixture disclosed herein. The cell suspension can be substantially free of culture medium serum-derived proteins. The cells can be suspended in culture medium (e.g., serum-free culture medium) or in a pharmaceutically acceptable carrier solution (e.g., sterile water or physiological saline). The devices also can have a hypodermic needle affixed to the orifice that communicates with the syringe chamber.

The invention also provides methods for making the devices described above. The methods can involve preparing a cell suspension using any of the methods described herein, and then placing the cell suspension in the syringe chamber of a syringe.

The invention will be further described in the following examples, which do not limit the scope of the invention described in the claims.

### EXAMPLES

### Example 1 - Isolation of autologous UMC and fibroblasts

Cells were harvested and enriched in vitro by initiation of cultures from a skin biopsy obtained from a normal healthy human volunteer as follows. Biopsies of about 3 to 5 mm³ were obtained from the post auriculum area, and fibroblast tissue culture initiated as described above using DMEM containing 4500 mg/L D-glucose, 2 mM L-glutamine, nonessential amino acids, and 10% FBS. Colonies of non-adherent, actively growing cells were observed after adherent fibroblasts had reached full confluence in passage two or three. This process could be shortened by initiation of the culture in low serum and by the presence of 5 ng/ml aFGF, or by growth of the cells in a plasma clot (see Example 2) with addition of 300 mM CaCl₂ to a final concentration of 15 to 30 mM. Each colony contained between 2 and about 80 cells that resembled epithelioid cells and were actively dividing. The colonies were collected by aspiration of culture medium containing the floating colonies and centrifugation of this medium. The cells pelleted by centrifugation were transferred to new tissue culture vessels by direct seeding in fresh culture medium containing aFGF and heparin (DMEM containing 4500 mg/L D-glucose, 2 mM L-glutamine, 2.5% heat inactivated FBS, 5 ng/mL recombinant human aFGF, and 5 *µ*g/mL heparin). The cell suspension was added to fresh tissue culture flasks, which were incubated at 37°C. Cells were fed twice weekly, and were passaged or differentially trypsinized when confluence was reached (generally within one to two weeks). Colonies of cobblestone-like cells were observed within about 3-6 weeks of initiation of the culture. Isolation of the colonies and culturing in fresh tissue culture vessels caused the cells to become adherent.

Colonies of non-adherent cells also were isolated from human adipose tissue as follows. The tissue was cut into small pieces and all visible membranes were removed. The tissue was placed in culture in DMEM containing 4500 mg/L D-glucose, 2 mM L-glutamine, 2.5% heat inactivated FBS, 1 to 10 ng/mL recombinant human aFGF, and 5 *µ*g/mL heparin. Under these conditions, cobblestone-like cells were actively shed from the adipose tissue, and continued to grow for a prolonged period of time. The pieces of adipose tissue were washed and placed into fresh tissue culture vessels. Within about 2 weeks, UMC were isolated from the tissue by treatment with collagenase IV for about 5-15 minutes at 37°C. New cells from the adipose tissue remained actively growing in culture for over a year, until the cultures were terminated. Once the cultures were fully grown, clusters of non-adherent cells were observed. When these cells were reseeded in fresh tissue culture flasks, the same type of cells were observed to be actively growing.

In the presence of aFGF, cells in the cultures from both skin and adipose tissue were morphologically homogeneous in appearance, resembling epithelioid like cells with a cobblestone-like morphology. Upon removal of aFGF from the culture medium, however, most of the cells fully differentiated into adherent fibroblasts. The cobblestone-like non-adherent cells also were observed in cultures initiated from bone marrow, using a method described by Marko et al. (supra). It is concluded that at least a subpopulation of the non-adherent epithelioid-like cells harvested from fibroblast cultures established from dermis or from cultures of adipose tissue or bone marrow are UMC.

### Example 2 - Isolation and growth of cells in plasma clots

Cells isolated as described herein are cultured in plasma clot gels. This method is useful for isolating and purifying various cell types. Plasma clots are prepared using autologous human plasma, bovine plasma, or calf plasma, with fibrin or calcium chloride used as the clotting agent. Plasma clot gels are prepared, prewashed, and pre-incubated, and then seeded with the desired cell types transferred directly from a culture flask. Colonies of cells are readily isolated directly from the clot gel. Alternatively, cells are isolated by cloning devices or by well plate inserts, in which case cells are seeded into inserts and support cells (e.g., fibroblasts or other desired cell types) for growth factor delivery are seeded below the inserts.

### Example 3 - Treatment of burn wounds with fibroblast suspensions

A patient with a six-month old, non-healing burn wound covering the entire forehead was treated by directly injecting into the wound a suspension of about 30 to 40x10⁶ fibroblasts per ml in phenol red-free DMEM. The burn wound was similarly injected twice more at two week intervals, although by the second injection the wound was almost completely closed. The wound subsequently healed with a minimum of scarring. Other patients with laser bums were treated with similar success. After three injections, unused cells were stored in liquid nitrogen for future use if necessary. Compositions containing keratinocytes as well as fibroblasts will be at least as effective, and probably more effective, as those containing fibroblasts alone. Moreover, in view of the fact that UMC can differentiate into fibroblasts they will be as effective as fibroblasts, whether administered without or with fibroblasts.

### Example 4 - Optimizing the treatment of UMC with activating factors

In vitro experiments are carried out to determine the optimal concentration of activating compound to add to a UMC culture. UMC cultured either in matrices or in monolayers are incubated with various concentrations of activating compounds such as those disclosed herein. The optimal concentration is determined by assessing which concentration is associated with the greatest level of cell proliferation. Alternatively, the optimal concentration is that which results in the greatest level of collagen production. Collagen is secreted into the culture medium, and is measured by immunological assays such as western blotting or ELISA, for example. The optimal concentrations of activating compounds tested with fibroblasts were 10⁻⁵ M for ascorbic acid, 10⁻⁷ M for ascorbyl palmitate, and 10⁻⁶- 10⁻⁷ M for L-lipoic acid.

### OTHER EMBODIMENTS

It is to be understood that while the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. Use of undifferentiated mesenchymal cells (UMCs) for the manufacture of a medicament for use in the treatment of tissue degeneration or a tissue defect selected from soft tissue defect, a defect of an oral mucosa, trauma to an oral mucosa, periodontal disease, diabetes, a cutaneous ulcer and venous stasis, in which the medicament comprises autologous, passaged UMCs substantially free of culture medium serum-derived proteins.

2. The use as claimed in claim 1, wherein said soft tissue defect is selected from a facial depression, underdevelopment of the breast, absence of breast, a vocal cord defect, velopharyngeal incompetence, Poland's syndrome, underdevelopment of the penis, hypoplasia of the lips, and a subcutaneous atrophy or muscular atrophy.

3. The use as claimed in claim 1, wherein said trauma to an oral mucosa is an extraction socket resulting from an extraction of a tooth.

4. The use as claimed in claim 1, wherein said periodontal disease comprises periodontal degeneration, gingivitis, or a non-healing wound of a palatal mucosa or a gingival mucosa.

5. The use as claimed in claim 1, wherein said tissue defect is hypoplasia of a lip or a lip fold.

6. The use as claimed in any one of claims 1 to 5, wherein said autologous UMC are from gums, palate, skin, lamina propria, connective tissue, fascia, bone marrow, or adipose tissue of said subject.

7. The use as claimed in any one of claims 1 to 6, wherein said composition further comprises a biodegradable matrix, wherein said UMC are integrated within or on said matrix.

8. The use as claimed in any one of claims 1 to 7, wherein said composition further comprises a biodegradable acellular filler.

9. The use as claimed in any one of claims 1 to 8, wherein said composition further comprises autologous, passaged fibroblasts.

10. The use as claimed in any one of claims 1 to 9, wherein said composition comprises a pharmaceutically acceptable carrier.

11. The use as claimed in any one of claims 1 to 10, wherein said tissue defect or tissue degeneration is a dental or periodontal defect or a bone defect.

12. The use as claimed in any one of claims 1 to 11, wherein said composition is for injecting into tissue adjacent to said site of tissue defect or degeneration.

13. The use as claimed in any one of claims 1 to 12, wherein said composition is substantially free of cells other than passaged autologous UMC, passaged, autologous fibroblasts and, optionally, passaged, autologous keratinocytes.

14. Use of undifferential mesenchymal cells for the manufacture of a medicament for simultaneous, sequential or separate administration of for use in the repair of tissue that has degenerated in a subject as a result of a disease, disorder, or defect in said subject, wherein said UMC are passaged UMC substantially free of culture medium serum-derived proteins; and said fibroblasts are autologous, passaged fibroblasts into substantially free of culture medium serum-derived proteins.

15. A method for repairing tissue in a subject, wherein said method comprises:
(a) providing a composition comprising autologous, passaged UMC, wherein said composition is substantially free of culture medium serum-derived proteins;
(b) identifying a site of tissue defect or tissue degeneration in said subject; and
(c) placing said composition at said site so that said tissue defect or degeneration is repaired;
wherein said tissue defect or a degeneration is a cosmetic defect of the skin.

16. Use of undifferentiated mesenchymal cells for the manufacture of an agent for use in the treatment of a cosmetic or aesthetic defect of the skin of a subject, in which the agent comprises autologous, passaged UMC substantially free of culture medium serum-derived proteins.

17. Use as claimed in claim 16, wherein said agent is for injecting into tissue adjacent to the site of said defect of the skin.

18. The method as claimed in claim 15 or the use as claimed in claim 16 or 17, wherein said cosmetic defect is a rhytid, a depressed scar, a cutaneous depression of non-traumatic origin, a laugh line, a stretch mark, a wrinkle, a wound scar, an acne scar, or subcataneous atrophy.

19. The method or use as claimed in any one of claims 15 to 18, wherein said autologous UMC are from gums, palate, skin, lamina propria, connective tissue, fascia, bone marrow, or adipose tissue of said subject.

20. The method or use as claimed in any one of claims 15 to 19, wherein said composition further comprises a biodegradable matrix, wherein said UMC are integrated within or on said matrix.

21. The method or use as claimed in any one of claims 15 to 20, wherein said composition further comprises a biodegradable acellular filler.

22. The method or use as claimed in any one of claims 15 to 21, wherein said composition further comprises autologous, passaged fibroblasts.

23. A composition for use as a medicament, wherein said composition comprises autologous, passaged UMC and autologous, passaged fibroblasts, and wherein said composition is substantially free of culture medium serum-derived proteins.

24. A composition for the cosmetic repair of tissue, wherein said composition comprises autologous, passaged UMC and autologous, passaged fibroblasts, and wherein said composition is substantially free of culture medium serum-derived proteins.

25. A composition as claimed in claim 23 or claim 24 wherein said composition is substantially free of cells other than autologous, passaged UMC, autologous, passaged fibroblasts and, optionally autologous, passaged keratinocytes.

26. The composition as claimed in any one of claims 23 to 25, wherein said autologous fibroblasts are from gums, palate, skin, lamina propria, connective tissue, bone marrow, fascia, or adipose tissue of said subject.

27. The composition as claimed in any one of claims 23 to 26, wherein said UMC are from dermal tissue, adipose tissue, connective tissue, fascia, lamina propria, or bone marrow.

28. The composition as claimed in any one of claims 23 to 27, wherein said UMC or said fibroblasts have been treated with one or more activating compounds.

29. The composition as claimed in claim 28, wherein said one or more activating compounds is selected from ascorbyl palmitate, linoleic acid, quinic acid, quinic acid salts, quinic lactones, CoEnzyme Q-10, L-hydroxy acid, L-lipoic acid, calcium monophosphate, calcium triphosphate, niacin, dehydroepiandrosterone (DHEA), dimethylamino ethanol (DMAE), α-tocopherol, a bone morphogenic protein (BMP), vitamin E, vitamin C, carotenoids, glycolic acid, carboxyalkyl esters, estriol, acetyl-L-camitine, deprenyl, lycopene, nicotinamide adenine dinucleotide (NADH), cysteine, procysteine, pikamilon, vinpocetine, retinoic acid, antineoplastons, and growth factors.

30. The composition as claimed in any one of claims 23 to 29, wherein said UMC or said fibroblasts have been exposed to a low energy laser light.

31. The composition as claimed in any one of claims 23 to 30, wherein said composition further comprises a biodegradable matrix, wherein said UMC and said fibroblasts are integrated within or on said matrix.

32. The composition as claimed in claim 31, wherein said matrix, prior to combination with said UMC and said fibroblasts, comprises one or more substances selected from collagen, glycosaminoglycans, gelatin, polyglycolic acid, cat gut, demineralized bone, hydroxyapatite, and anorganic bone.

33. The composition of any one of claims 23 to 32, further comprising a biodegradable acellular injectable filler.

34. The composition of claim 33, wherein said biodegradable acellular injectable filler, prior to combination with said UMC, comprises one or more substances selected from: (a) an injectable dispersion of autologous collagen fibers; (b) collagen; (c) solubilized gelatin; (d) solubilized polyglycolic acid; (e) solubilized cat gut; (f) porcine gelatin powder and amino caproic acid dispersed in sodium chloride solution and an aliquot of plasma from said subject; and (g) hyaluronic acid.

35. A method for making a cellular composition for repairing tissue, said method comprising:
(a) separating starting cells from a biopsy of UMC-containing tissue obtained from a subject;
(b) culturing said starting cells; and
(c) harvesting a population of non-adherent derivative cells from said culture,
wherein said non-adherent derivative cells comprise UMC.

36. The method as clamed in claim 35, further comprising one or more rounds of derivitization comprising repeating steps (c) and (d) utilizing the harvested population of non-adherent derivative cells from the previous round as the starting cells.

37. The method as claimed in claim 36, wherein said one or more additional rounds of derivatization comprises from one to twenty rounds.

38. The method as claimed in any one of claims 35 to 37, wherein said UMC-containing tissue is dermal tissue, adipose tissue, connective tissue, fascia, lamina propria or bone marrow.

39. The method as claimed in any one of claims 35 to 38, further comprising culturing said non-adherent cells in the presence of acidic fibroblast growth factor.

40. A method for making a composition for repairing tissue that has degenerated in a subject as a result of a disease, disorder, or defect in said subject, said method comprising:
(a) separating autologous UMC from a biopsy of UMC-containing tissue obtained from said subject;
(b) culturing said autologous UMC under conditions that produce autologous UMC that are substantially free of culture medium serum-derived proteins;
(c) exposing said cultured autologous UMC to conditions that result in suspension of said UMC;
(d) separating autologous fibroblasts from a biopsy of fibroblast-containing tissue obtained from said subject;
(e) culturing said autologous fibroblasts under conditions that produce fibroblasts that are substantially free of culture medium serum-derived proteins;
(f) exposing said cultured autologous fibroblasts to conditions that result in suspension of said fibroblasts; and
(g) mixing said UMC suspension with said fibroblast suspension.

41. The method as claimed in claim 40, wherein said UMC-containing tissue is selected from gums, palate, skin, lamina propria, connective tissue, fascia, bone marrow, and adipose tissue.

42. The method as claimed in claim 40 or 41, wherein said autologous fibroblast-containing tissue is selected from gums, palate, skin, lamina propria, connective tissue, fascia, bone marrow, and adipose tissue.

43. The method as claimed in any one of claims 40 to 42, wherein prior to said combining, said UMC or said fibroblasts are exposed to one or more activating compounds.

44. The method as claimed in claim 43, wherein said one or more activating compounds is selected from ascorbyl palmitate, linoleic acid, quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, a BMP, vitamin E, vitamin C, carotenoids, glycolic acid, carboxyalkyl esters, estriol, acetyl-L-camitine, deprenyl, lycopene, NADH, cysteine, procysteine, pikamilon, vinpocetine, retinoic acid, antineoplastons, and growth factors.

45. The method as claimed in any one of claims 40 to 44, wherein said UMC or said fibroblasts are exposed to a low energy laser light.

46. A method for making a composition for repairing tissue that has degenerated in a subject as a result of a disease, disorder, or defect in said subject, wherein said method comprises:
(a) providing autologous, passaged UMC;
(b) providing autologous, passaged fibroblasts;
(c) providing a biodegradable acellular matrix; and
(d) incubating said UMC and said fibroblasts with said biodegradable acellular matrix such that said UMC and/or fibroblasts integrate on or within said biodegradable acellular matrix, wherein said incubation results in a composition for repairing tissue, and wherein the conditions of said incubation are such that said composition is substantially free of culture medium serum-derived proteins.

47. The method as claimed in claim 46, wherein the step of providing autologous, passaged UMC comprises:
(a) separating autologous UMC from a biopsy of UMC-containing tissue obtained from said subject;
(b) culturing said UMC; and
(c) exposing said cultured UMC to conditions that result in suspension of said UMC.

48. The method as claimed in claim 46 or 47, wherein said UMC-containing tissue is selected from gums, palate, skin, lamina propria, connective tissue, fascia, bone marrow, and adipose tissue.

49. The method as claimed in any one of claims 46 to 48, wherein said biodegradable acellular matrix, prior to combination with said UMC and said fibroblasts, comprises one or more substances selected from collagen, glycosaminoglycans, gelatin, polyglycolic acid, cat gut, demineralized bone, hydroxyapatite, coral, and anorganic bone.

50. The method as claimed in any one of claims 46 to 48, wherein the step of providing autologous, passaged fibroblasts comprises:
(a) separating autologous fibroblasts from a biopsy of fibroblast-containing tissue obtained from said subject;
(b) culturing said fibroblasts; and
(c) suspending said fibroblasts.

51. The method as claimed in claim 50, wherein said fibroblast-containing tissue is selected from gums, palate, skin, lamina propria, connective tissue, fascia, bone marrow, and adipose tissue.

52. The method as claimed in any one of claims 46 to 51, wherein prior to said incubating with said biodegradable acellular matrix, said UMC or said fibroblasts are exposed to one or more activating compounds.

53. The method as claimed in claim 52, wherein said one or more activating compounds is selected from ascorbyl palmitate, linoleic acid, quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, a BMP, vitamin E, vitamin C, carotenoids, glycolic acid, carboxyalkyl esters, estriol, acetyl-L-camitine, deprenyl, lycopene, NADH, cysteine, procysteine, pikamilon, vinpocetine, retinoic acid, antineoplastons, and growth factors.

54. The method as claimed in any one of claims 46 to 53, wherein said UMC or said fibroblasts are exposed to a low energy laser light.

55. A method for making a composition for repairing tissue that has degenerated in a subject as a result of a disease, disorder, or defect in said subject, wherein said method comprises:
(a) providing autologous, passaged UMC, wherein said UMC are substantially free of culture medium serum-derived proteins;
(b) providing autologous, passaged fibroblasts, wherein said autologous, passaged fibroblasts are substantially free of culture medium serum-derived proteins;
(c) providing a biodegradable acellular filler; and
(d) combining said autologous, passaged UMC, said autologous, passaged fibroblasts, and said biodegradable acellular filler.

56. The method as claimed in claim 55, wherein the step of providing autologous, passaged UMC comprises:
(a) separating autologous UMC from a biopsy of UMC-containing tissue from obtained from said subject;
(b) culturing said autologous UMC under conditions that result in UMC that are substantially free of culture medium serum-derived proteins; and
(c) exposing said cultured UMC to conditions that result in suspension of said UMC.

57. The method as claimed in claim 56, wherein said UMC-containing tissue is selected from gums, palate, skin, lamina propria, connective tissue, fascia, bone marrow, and adipose tissue.

58. The method as claimed in any one of claims 55 to 57, wherein said biodegradable acellular filler, prior to combination with said UMC and said fibroblasts, comprises one or more substances selected from the group consisting of: (a) an injectable dispersion of autologous collagen fibers; (b) collagen; (c) solubilized gelatin; (d) solubilized polyglycolic acid; (e) solubilized cat gut; (f) porcine gelatin powder and amino caproic acid dispersed in sodium chloride solution and an aliquot of plasma from said subject; and (g) hyaluronic acid.

59. The method of any one of claims 55 to 58, wherein the step of providing autologous, passaged fibroblasts comprises:
(a) separating autologous fibroblasts from a biopsy of fibroblast-containing tissue obtained from said subject;
(b) culturing said autologous fibroblasts under conditions that result in fibroblasts that are substantially free of culture medium serum-derived proteins; and
(c) exposing said incubated autologous fibroblasts to conditions that result in suspension of said fibroblasts.

60. The method as claimed in claim 59, wherein said fibroblast-containing tissue is selected from gums, palate, skin, lamina propria, connective tissue, fascia, bone marrow, and adipose tissue.

61. The method as claimed in any one of claims 55 to 60, wherein prior to said combining with said biodegradable acellular filler, said UMC or said fibroblasts are exposed to one or more activating compounds.

62. The method as claimed in claim 61, wherein said one or more activating compounds is selected from ascorbyl palmitate, linoleic acid, quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, a BMP, vitamin E, vitamin C, carotenoids, glycolic acid, carboxyalkyl esters, estriol, acetyl-L-camitine, deprenyl, lycopene, NADH, cysteine, procysteine, pikamilon, vinpocetine, retinoic acid, antineoplastons, and growth factors.

63. The method of any one of claims 55 to 62, wherein said UMC or said fibroblasts are exposed to a low energy laser light.

64. Use of autologous, passaged keratinocytes and autologous, passaged fibroblasts, for the manufacture of a medicament for use in the repair of a bum wound in a subj ect.

65. The use as claimed in claim 64, wherein said medicament further comprises autologous, passaged UMC.

66. The use as claimed in claim 64 or 65, wherein said medicament further comprises an enhancing compound.

67. The use as claimed in any one of claims 64 to 66, wherein said medicament is for injecting into said wound.

68. A method for making a cellular composition for repairing tissue, said method comprising:
(a) separating autologous keratinocytes from a biopsy of keratinocyte-containing tissue obtained from said subject;
(b) culturing said autologous keratinocytes under conditions that produce autologous keratinocytes that are substantially free of culture medium serum-derived proteins;
(c) exposing said keratinocytes to conditions that result in suspension of said keratinocytes;
(d) separating autologous fibroblasts from a biopsy of fibroblast-containing tissue obtained from said subject;
(e) culturing said autologous fibroblasts under conditions that produce autologous fibroblasts that are substantially free of culture medium serum-derived proteins;
(f) exposing said fibroblasts to conditions that result in suspension of said fibroblasts; and
(g) combining said suspended keratinocytes and said suspended fibroblasts.

69. The method as claimed in claim 68, wherein said keratinocyte-containing tissue is selected from skin, oral mucosa, gums, buccal tissue, esophageal tissue, exocervical tissue, and conjunctival tissue.

70. The method as claimed in claim 68 or 69, wherein said fibroblast-containing tissue is selected from gums, palate, skin, lamina propria, connective tissue, fascia, bone marrow, and adipose tissue.

71. The method as claimed in any one of claims 68 to 70, wherein prior to combining with said keratinocytes, said fibroblasts are exposed to one or more activating compounds.

72. The method of claim 71, wherein said one or more activating compounds is selected from ascorbyl palmitate, linoleic acid, quinic acid, quinic acid salts, quinic lactones, CoEnzyme Q-10, L-hydroxy acid, L-lipoic acid, calcium monophosphate, calcium triphosphate, niacin, DHEA, DMAE, α-tocopherol, a BMP, vitamin E, vitamin C, carotenoids, glycolic acid, carboxyalkyl esters, estriol, acetyl-L-carnitine, deprenyl, lycopene, NADH, cysteine, procysteine, pikamilon, vinpocetine, retinoic acid, antineoplastons, and growth factors.

73. The method as claimed in any one of claims 68 to 72, wherein said fibroblasts are exposed to a low energy laser light.

74. A method for repairing tissue in a subject, wherein said method comprises:
(a) providing a composition comprising autologous, passaged UMC, wherein said composition is substantially free of culture medium serum-derived proteins;
(b) identifying a site of tissue defect or tissue degeneration in said subject; and
(c) placing said composition at said site so that said tissue defect or degeneration is repaired;
wherein said tissue defect or degeneration comprises a soft tissue defect, a defect of an oral mucosa, trauma to an oral mucosa, periodontal disease, diabetes, a cutaneous ulcer, venous stasis, or a cosmetic defect of the skin.
